# EUROPEAN PATENT APPLICATION

(11) **EP 2 020 419 A1**
(43) Date of publication of application: **04.02.2009**
(21) Application number: 07075657.2
(22) Date of filing: 31.07.2007
(51) Int. Cl.: C07K 16/28, C07K 16/30, A61K 39/395

(54) **Anti ephB4 antibody fragments**

(71) Applicant: Bayer Schering Pharma Aktiengesellschaft, 13353 Berlin (DE); MorphoSys AG, 82152 Martinsried/München (DE)
(72) Inventor: Petrul, Heike, D-10249 Berlin (DE); Menrad, Andreas, Witchford, Elsy Cambs CB6 2 HP (GB); Willuda, Jörg, D-13127 Berlin (DE); Zopf, Dieter, D-14167 Berlin (DE); Glienke, jens, D-10405 Berlin (DE); Prassler, Josef, D-82110 Germering (DE); Popp, Andreas, D-86916 Kaufering (DE)

(57) **Abstract**

The invention comprises antibody fragments, specifically recognising the EphB4 receptor comprising a CDR of a heavy chain comprising CDR-H1, CDR-H2 and CDR-H3 and a light chain comprising CDR-L1, CDR-L2 and CDR-L3. The different CDR are characterised by concrete amino acid sequences of the hyper variable regions, the frame work regions and the variable regions.

The antibody fragments according to the invention are a medicament for use in pathological angiogenesis in particular cancer therapy.

## Description

### FIELD OF THE INVENTION

This invention relates generally to antibody fragments specifically recognizing the receptor EphB4 and to methods of inhibiting angiogenesis in a mammal using said antibody fragments.

### BACKGROUND OF THE INVENTION

The process of angiogenesis, by which new blood vessels develop from existing ones by branching and sprouting, is crucial for vascular remodeling in embryogenesis and normal tissue homeostasis (e.g., female reproductive cycle). Angiogenesis also plays an important role in the pathogenesis of diseases such as cancer and retinopathy. The pro-angiogenic environment of these diseases results in new blood vessels which allow the development and the maintenance of the pathological state.

The Eph family of receptor tyrosine kinases and their ligands, the ephrins, are critical regulators of vascular remodeling in the embryo, postnatal vascular remodeling, and tumor neovascularization. With its currently known 15 members, the Eph family represents the largest family of receptor tyrosine kinases and is divided into the A and the B classes based on sequence homology and the structural differences of the ligands. The membrane-attached ligands differ in the transduction of the signal into the cell: The A ephrins are linked to the cell via a glycosylphosphatidylinositol (GPI) anchor, whereas the B ephrins have a transmembrane region and a short intracellular domain.

The Eph receptors and ephrins mediate bi-directional signaling: forward signaling through the Eph receptor and reverse signaling via the ephrins ligand. In general, nine different EphA receptors (EphA1 - A9) promiscuously interact with six A-ephrins (Eph-rinA1 - A6?), whereas the receptors of the B -subclass (EphB1 - B6) interact with three different B-ephrins (ephrin B1 - B3). The EphB4 receptor ("EphB4"), however, only binds the ligand Ephrin B2, making this one-receptor-one-ligand interaction an interesting target for therapeutic approaches.

EphB4 and its ligand Ephrin B2 are expressed in the developing central nervous system, where they function in contact-mediated axon guidance, axon fasciculation, and guided cell migration. Ephrin B2 and EphB4 mediate spatial organization signalling during angiogenesis and vessel assembly. In other contexts, however, Eph/ephrin signalling appears to enhance cellular adhesion, such as in a human breast cancer model in mice (Noren et al., Proc. Natl. Acad. Sci. USA 101, 5583-88, 2004). In this model, EphB4 on the tumour cells attracts Ephrin B2-bearing endothelial cells. As a result, the tumour is better vascularized and therefore grows faster than the control. Interestingly, this model uses a kinase-deleted mutant of EphB4 which shows that the (indirect) growth advantage of the tumour cells is not dependent on the EphB4 kinase domain, but rather on the EphB4 ectodomain which interacts with Ephrin B2. In light of these results, blocking of the interaction between EphB4 and the ligand Ephrin B2 should lead to tumour growth inhibition. There is, therefore, a need in the art for molecules which can perform this function with high specificity and affinity.

The problem of the invention is to find an antibody fragment specifically recognising the EphB4 receptor with a high effectiveness. Further the antibody fragments can be used as a medicament especially for treatment of cancer, for example breast cancer.

Known IgG and IgM molecules (e.g. WO 2004/080425 A2) against EphB4 extracellular domain, cited in the literature of patents and science, are described vaguely are not available to the scientific community and are not described sufficiently for a teaching to create pre published state of the art. On page 63, line 5 to 11 the manufacture of anti EphB4 monoclonal antibodies is described which is an insufficient disclosure. Further on page 99 the use of anti EphB4 monoclonal antibodies are mentioned. In both text passages the amino acid sequences and / or the deposition of the monoclonal antibodies is lacking.

The problem is solved by an antibody fragment, specifically recognising the EphB4 receptor comprising a complementary determining region (CDR) of
a heavy chain
and
a light chain
wherein the CDR of the heavy chain (CDR-H) comprises the sequence of the amino acids of the
Seq Id No 1 (3640 CDR-H1) and
Seq Id No 2 (3640 CDR-H2) and
Seq Id No 3 (3640 CDR-H3)
or modifications thereof
or the
Seq Id No 7 (3639 CDR-H1) and
Seq Id No 8 (3639 CDR-H2) and
Seq Id No 9 (3639 CDR-H3)
or modifications thereof
or the
Seq Id No 13 (3641 CDR-H1) and
Seq Id No 14 (3641 CDR-H2) and
Seq Id No 15 (3641 CDR-H3)
or modifications thereof
and / or
wherein the CDR of the light chain (CDR-L) comprises the sequence of the amino acids of the
Seq Id No 4 (3640 CDR-L1) and
Seq Id No 5 (3640 CDR-L2) and
Seq Id No 6 (3640 CDR-L3)
or modifications thereof
or the
Seq Id No 10 (3639 CDR-L1) and
Seq Id No 11 (3639 CDR-L2) and
Seq Id No 12 (3639 CDR-L3)
or modifications thereof
or the
Seq Id No 16 (3641 CDR-L1) and
Seq Id No 17 (3641 CDR-L2) and
Seq Id No 18 (3641 CDR-L3)
or modifications thereof.

Further the problem is solved by an antibody fragment, specifically recognising the EphB4 receptor comprising a CDR of a heavy chain comprising CDR-H1, CDR-H2 and CDR-H3 and a light chain comprising CDR-L1, CDR-L2 and CDR-L3 wherein one of the heavy chains comprising
the CDR-H1 selected from group:
Seq Id No 1,
Seq Id No 7,
Seq Id No 13
or a modification thereof
and
the CDR-H2 selected from the group:
Seq Id No 2,
Seq Id No 8,
Seq Id No 14
and the CDR-H3 selected from the group:
Seq Id No 3,
Seq Id No 9,
Seq Id No 15
or modifications thereof
and / or
wherein one of the light chains comprising
the CDR-L1 selected from the group
Seq Id No 4,
Seq Id No 10,
Seq Id No 16
or modifications thereof
and
the CDR-L2 selected from the group
Seq Id No 5,
Seq Id No 11,
Seq Id No 17
or modifications thereof
and
the CDR-L3 selected from the group
Seq Id No 6,
Seq Id No 12,
Seq Id No 18
and modifications thereof.

Further the problem is solved by an antibody fragment specifically recognising the EphB4 receptor comprising
Seq Id No 19 (variable domain 3640 CDR-H) and
Seq Id No 20 (variable domain 3640 CDR-L)
or
Seq Id No 21 (variable domain 3639 CDR-H) and
Seq Id No 22 (variable domain 3639 CDR-L)
or
Seq Id No 23 (variable domain 3641 CDR-H) and
Seq Id No 24 (variable domain 3641 CDR-L)
or modifications thereof.

Further the problem is solved by an antibody fragment specifically recognising the EphB4 receptor comprising a heavy chain selected from the group:
Seq Id No 19 (3640 variable domain of CDR-H) and
Seq Id No 21 (3639 variable domain of CDR-H) and
Seq Id No 23 (3641 variable domain of CDR-H)
and modifications thereof,
and a light chain selected from the group:
Seq Id No 20 (3640 variable domain of CDR-H) and
Seq Id No 22 (3639 variable domain of CDR-H) and
Seq Id No 24 (3641 variable domain of CDR-H)
and modifications thereof.

Further the problem is solved by an antibody fragment specifically recognising the EphB4 receptor comprising
Seq Id No 25 (3640 CDR-H/C)
Seq Id No 26 (3640 CDR-UC)
or
Seq Id No 27 (3639 CDR-H/C)
Seq Id No 28 (3639 CDR-UC)
or
Seq Id No 29 (3641 CDR-H/C)
Seq Id No 30 (3641 CDR-L/C)
or modifications thereof.

Further the problem is solved by an antibody fragment specifically recognising the EphB4 receptor comprising heavy chain (CDR-H/C) selected from the group:
Seq Id No 25 (3640)
Seq Id No 27 (3639)
Seq Id No 29 (3641)
and
a light chain (CDR-UC) selected from the group:
Seq Id No 26 (3640)
Seq Id No 28 (3639)
Seq Id No 30 (3641)
and modifications thereof.

### Dependencies of the sequences:

The sequences of the invention are dependent. (In case of a wrong or questionable position number, the comparison of the concrete sequences is only relevant.) The sequences with the Seq ID No 19 - 24 comprises the three hypervariable regions each of the heavy and alternatively of the light chain.
Seq Id No 19 (3640 variable domain of CDR-H) comprises Seq Id No 1 from position 26 to 35, further Seq Id No 2 from position 47 to 66, further Seq Id No 3 from position 99 to 115;
Seq Id No 20 (3640 variable domain of CDR-H) comprises Seq Id No 4 from position 23 to 33, further Seq Id No 5 from position 45 to 55, further Seq Id No 6 from position 88 to 97;
Seq Id No 21 (3639 variable domain of CDR-H) comprises Seq Id No 7 from position 26 to 35, further Seq Id No 8 from position 50 to 66, further Seq Id No 9 from position 99 to 111;
Seq Id No 22 (3639 variable domain of CDR-H) comprises Seq Id No 10 from position 23 to 33, further Seq Id No 11 from position 45 to 55, further Seq Id No 12 from position 88 to 97;
Seq Id No 23 (3641 variable domain of CDR-H) comprises Seq Id No 13 from position 26 to 35, further Seq Id No 14 from position 47 to 66, further Seq Id No 15 from position 99 to 115;
Seq Id No 24 (3641 variable domain of CDR-H) comprises Seq Id No 16 from position 24 to 34, further Seq Id No 17 from position 46 to 56, further Seq Id No 18 from position 89 to 96;

The sequences with the Seq ID No 25 - 30 comprises the three hypervariable regions each of the heavy and alternatively of the light chain each of the chain connected with a constant region .
Seq Id No 25 (3640 CDR-H/C) comprises Seq Id No 1 from position 26 to 35, further Seq Id No 2 from position 47 to 66, further Seq Id No 3 from position 99 to 115;
Seq Id No 26 (3640 CDR-UC) comprises Seq Id No 4 from position 23 to 33, further Seq Id No 5 from position 45 to 55, further Seq Id No 6 from position 88 to 97;
Seq Id No 27 (3639 CDR-H/C) comprises Seq Id No 7 from position 26 to 35, further Seq Id No 8 from position 50 to 66, further Seq Id No 9 from position 99 to 111;
Seq Id No 28 (3639 CDR-UC) comprises Seq Id No 10 from position 23 to 33, further Seq Id No 11 from position 45 to 55, further Seq Id No 12 from position 88 to 97;
Seq Id No 29 (3641 CDR-H/C) comprises Seq Id No 13 from position 26 to 35, further Seq Id No 14 from position 47 to 66, further Seq Id No 15 from position 99 to 115;
Seq Id No 30 (3641 CDR-UC) comprises Seq Id No 16 from position 24 to 34, further Seq Id No 17 from position 46 to 56, further Seq Id No 18 from position 89 to 96.

Further the sequences Seq Id No 25 - 30 comprise the sequences of the combined hypervariable regions and the framework (Seq Id No 19 to 24) in the concrete situation:
Seq Id No 25 (3640 CDR-H/C) comprises Seq Id No 19 from position 1 to 126;
Seq Id No 26 (3640 CDR-UC) comprises Seq Id No 20 from position 1 to 110;
Seq Id No 27 (3639 CDR-H/C) comprises Seq Id No 21 from position 1 to 122;
Seq Id No 28 (3639 CDR-UC) comprises Seq Id No 22 from position 1 to 110;
Seq Id No 29 (3641 CDR-H/C) comprises Seq Id No 23 from position 1 to 126;
Seq Id No 30 (3641 CDR-UC) comprises Seq Id No 24 from position 1 to 109.

### ADVANTAGES

Antibodies against the EphB4 receptor are known and described in the scientific and /or patent literature. The disadvantage of the antibodies of this state of the art is that the function of the antibodies is described, but the sequence is not mentioned, further the antibodies are not available, because they are not deposited in any cell culture collection. Therefore, the pre - published antibodies lack the technical teaching. The structure is not reproducible. The antibodies, described in the literature are NOT commercially available.

Commercial available antibodies against the EphB4 receptor are known, but only de-cribed concerning the binding function. Further, features especially function *in vivo* are not disclosed. Although the antibodies are commercial available, the amino acid sequences are not described anywhere. the preferred technique to analyse the amino acid sequence, is the Edman degradation. The analysis is an undue burden for a scientist to analyse more than 1000 amino acids. Further, the normal analysis error will make the data insecure concerning the exact amino acid sequence, necessary for a state of the are fulfilling the requirement of reproducibility.

The antibody fragments of the invention have instead the advantage of being characterised by clear technical features, like sequence and function.

Further it is made obvious that the monomers (for example Fab') and the dimers (for example F(ab')₂) block the interaction of the EphrinB2 on for example endothelial cells. Wherein, only the dimeric form induces the kinase activity of the intracellular part. These two different functions can only be found by sequences according to the invention, because for the first time monomers and dimers with known sequences can be compared, especially two known light or heavy chains can be combined to form one monomer.

### FURTHER EMBODIMENTS

A further embodiment of the invention is an antibody fragment which exhibits a substantially equivalent antigen binding to the EphB4 receptor comparable to an antibody fragment according to the invention claims
or
an antibody fragment which binds to the same epitope on the EphB4 receptor like an antibody fragment according to the invention.

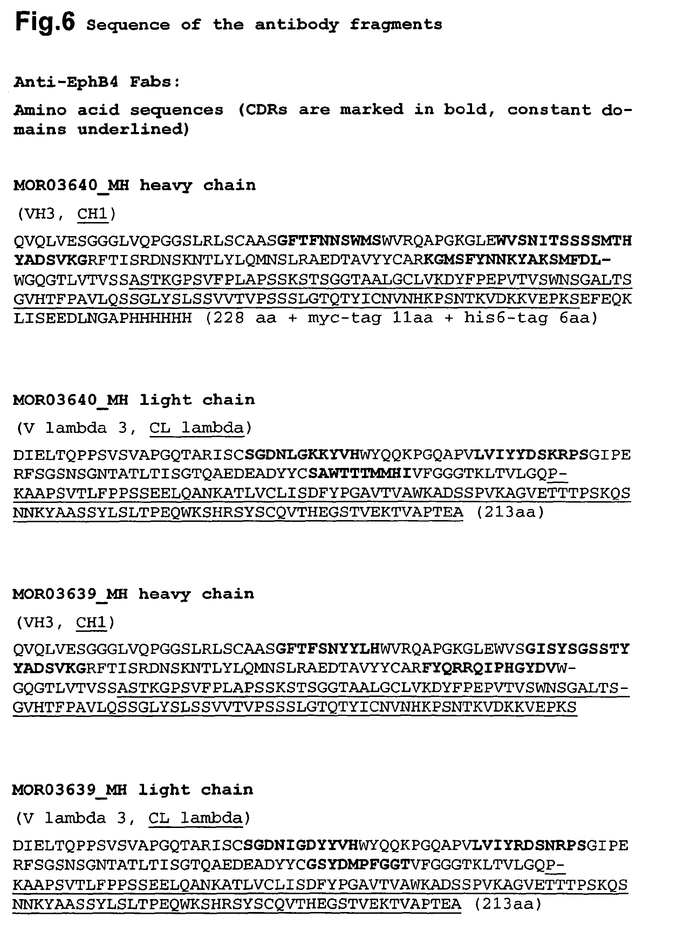

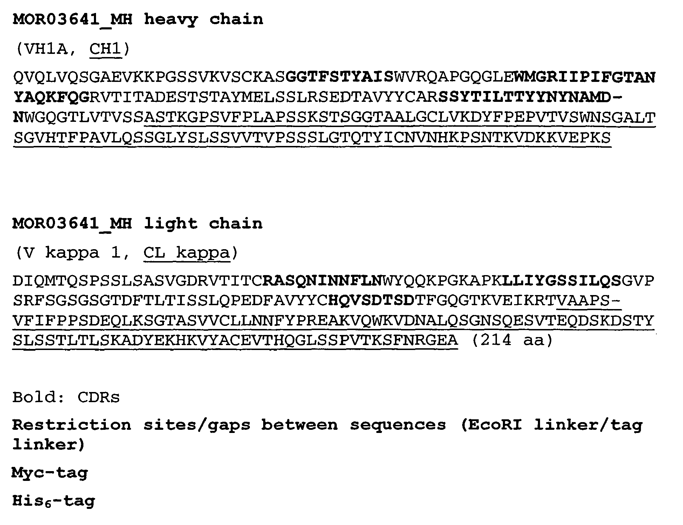

Substantially equivalent antigen binding or the same epitope binding can be measured by competitive inhibition kinetic of one of the claimed and defined antibody parts of the invention, especially the concrete antibody fragments of Fig. 6. Further the inhibition of the phosphorylation by the monomeric antibody fragments (see example 3) or the phosphorylation by the dimeric antibody fragments (see example 4) are indicators for substantially equivalent antigen binding or the same epitope binding. Other techniques to show competitive binding of anti-EphB4 antibody and Ephrin B2 ligand include competitive ELISA or Biacore^{™}.

The heavy and the light chain can be combined by dimerisation. Dimerisation tools are well described in the literature. Examples of these dimerisation tools are peptide linker, disulfide bridges, double helices. Typical realisations are Fab' (here in this application called monomers, because comprising the variable part and the constant part of the heavy AND the light chain) and F(ab')₂ (here in this application called dimer, because comprising two realisations of the variable part and the constant part of the heavy AND the light chain, connected in this concrete situation by the dimerisation tool of disulfide bridges)

If the dimerisation tool is a peptide antibody - linker the heavy chain might be N-terminal and the light chain might be C-terminal or
the heavy chain might be C-terminal and the light chain might be N-terminal.

The sequence of the antibody linker is described in the literature (see EP 0 573 551; EP 0 318 554 and EP 0 623 679).

The antibody fragment according to the invention can be used in pathological angiogenesis in particular cancer therapy, but also e.g. for the treatment of diabetic retinopathy.

The antibody fragment according to the invention can be combined with a further substance in cancer therapy.

Further the invention comprises a DNA coding for
antibody fragments, specifically recognising the EphB4 receptor
wherein the amino acid sequence comprises combinations and sequences as mentioned in the invention before.

Additionally, the invention provides human antibody fragments which bind to the extracellular domain of EphB4. Preferred antibodies were identified by screening a synthetic phage display antibody library (HuCAL®; Knappik et al., J. Mol. Biol 296, 57-86, 2000). Antibody fragments of the invention can be used to inhibit binding of Ephrin B2 to EphB4, thereby inhibiting activities mediated by EphB4 and by Ephrin B2.

The antibody fragments are useful for a variety of purposes, particularly for treating diseases characterized by abnormal angiogenesis, e.g. an increased angiogenesis, such as cancer. Preferably the antibody fragments of the invention are capable of modulating, e.g. inhibiting or stimulating EphB4 phosphorylation *in vivo.* Phosphorylation of EphB4 can be detected using any suitable means known in the art. *See, e.g*., Noren *et al.,* 2005, or Palmer et al., Mol Cell. 2002 Apr; 9(4)725-37. WO 04/080425 describes a cell-based EphB4 tyrosine kinase assay.

Further, the present invention refers to antibody fragments which bind to the same epitope on the EphB4 receptor as one of the antibody fragments MOR 03639, MOR 03640 or MOR 03641. The epitopes to which the antibody fragments bind may be determined by cross blocking and/or epitope mapping as known in the art.

The antibody fragments specifically recognize the EphB4 receptor, particularly the human EphB4 receptor. An ELISA assay can be carried out to identify antibodies which cross-react with human and, e.g., mouse EphB4 with high affinity and specificity. Functional cross-reactivity can be confirmed using, for example, a phosphorylation assay. Therapeutic utility of the antibody fragments can then be tested *in vivo* in an animal model, such as the mouse model described in the examples.

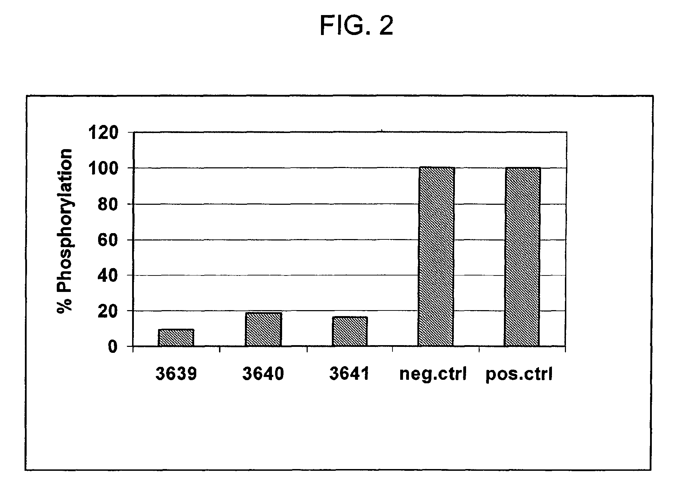

Antibody fragments of the invention bind to an epitope of EphB4 and preferably interfere with its binding to Ephrin B2. This interference can inhibit EphB4-mediated signalling. Monomeric anti-EphB4 antibody fragments invention block EphrinB2-induced phosphorylation of EphB4, which will block the receptor's kinase activity (see FIG. 2).

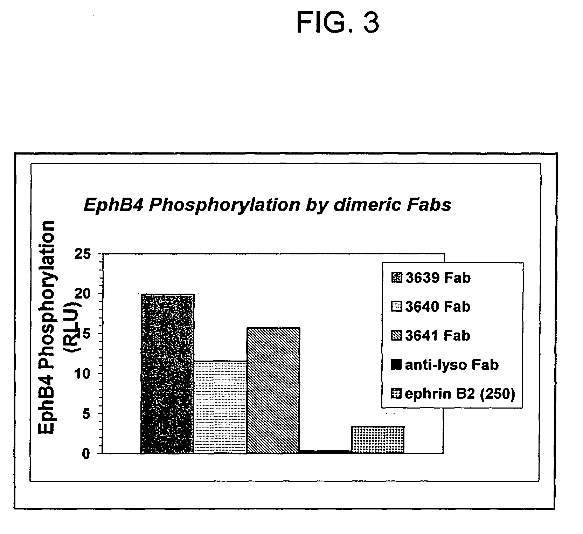

Other antibody fragments stimulate phosphorylation of EphB4 (See FIG. 3).

### DEFINITIONS:

A **combination** is a pharmaceutical composition comprising at least two pharmaceutically effective compounds. These compounds can be administered simultaneously or one after the other with a time gap of 1 minutes to 1 day. The compounds can be administered via the same route or in different manners. So the parallel oral and parenteral administration are possible.

A **composition** comprises at least one pharmaceutically effective compound and further pharmaceutically acceptable diluents and carrier.

A **fusion protein** is characterised by a protein comprising different amino acid sequences which are defined by the origin and/or by special functions.

**In the former literature epitopes of EphB4 specifically recognised by antibodies are not described.**

**Antibody linker** are described in EP 0 573 551; EP 0 623679 and EP 0 318554, which documents are introduced by reference.

**Fusion protein linker** are described in EP 0 573 551; EP 0 623679 and EP 0 318554, which documents are introduced by reference.

**Further substances used in cancer therapy** are described in WO2005/108415 (Applicant: BIOGEN IDEC MA INC. Filing date: 02. May 2005), exemplary cytotoxins include, in general, cytostatic agents, alkylating agents, antimetabolites, anti-proliferative agents, tubulin binding agents, hormones and hormone antagonists, and the like. Exemplary cytostatics that are compatible with the present invention include alkylating substances, such as mechlorethamine, triethylenephoramide, cyclophosphamide, ifos-famide, chlorambucil, busulfan, melphalan or triaziquone, also nitrosourea compounds, such as carmustine, lomustine, or semustine. Other preferred classes of cytotoxic agents include, for example, the maytansinoid family of drugs. Other preferred classes of cytotoxic agents include, for example, the anthracycline family of drugs, the vincy drugs, the mitomycins, the bleomycins, the cytotocix nucleosides, the pteridine family of drugs, diynenes, and the podophyllotoxins. Particularly useful members of those classes include, for example, adriamycin, carminomycin, daumorubicin (daunomycin) doxorubicin, aminopterin, methotrexate, methopterin, mithramycin, streptonigrin, di-chloromethotrexate, mitoycin C, actinomycin-D, porfiromycin, 5-fluorouracil, floxuridine, ftorafur, 6-mercaptopurine, cytarabine, cytosine arabinoside, podophyllotoxin, or podophyllotoxin derivatives such as etoposide or etoposide phosphate, melphalan, vin-blastine, vincristine, leurosidine, vindesine, leurosine and the like. Still other cytotoxins that are compatible with the teachings herein include taxol, taxane, cytochalasin B, gramicidin D, ethidium bromide, emetine, tenoposide, colchicin, dihydroxy anthracin dione, mitoxantrone, procaine, tetracaine, lidocaine, propranolol, and puromycin and analogs or homologs thereof. Hormones and hormone antagonists, such as corticosteroids, e.g. prednisone, progestins, e.g. hydroxyprogesterone or medroprogesterone, es-trogens, e.g. diethylstilbestrol, antiestrogens, e.g. tamoxifen, androgens, e.g. testosterone, and aromatase inhibitors, e.g. aminogluthetimide are also compatible with the teachings herein. One skilled in the art may make chemical modifications to the desired compound in order to make reactions of the compound more convenient for purposes of preparing combination of the invention.

**"Antibody fragments" comprise** a portion of an intact antibody, preferably comprising the antigen-binding or variable region thereof. Examples of antibody fragments include Fab, Fab', F(ab')₂ and Fv fragments; diabodies; linear antibodies; single-chain antibody molecules; and multispecific antibodies formed from antibody fragments. Examples are antibodies, native antibodies, Fab - fragments, Fab' (here defined a monomers) and F(ab')₂ (here defined as dimers), "Fv", Single-chain Fv" or' scFv" antibody fragments; monoclonal antibody; monoclonal antibodies" isolated from phage antibody libraries; chimeric antibodies; humanised antibodies; antibody fragments isolated from antibody phage libraries, human antibodies.

Monomeric antibody fragments, for example Fab fragments, are preferred. Monomeric fragments have several advantages over full IgG molecules. For example, they can better penetrate tumours and are cleared more quickly than full IgG molecules. In addition, Fab fragments can be conveniently and inexpensively produced in E. coli, considerably lowering the cost of producing the fragments. The Fab fragments described in this disclosure are easily amenable to further genetic modification. This genetic engineering includes, but is not limited to, affinity maturation of the antibody fragments. If desired, however, EphB4 Fabs can be converted into full immunoglobulins, for example IgG1 antibodies. Method of carrying out such conversions are well known in the art. Other modifications include PEGylation to change pharmacodynamics of proteins. PEGylation is offered by commercial providers, e.g. Celares (www.celares.com)

**Antibody fragments:** Various techniques have been developed for the production of antibody fragments. Traditionally, these fragments were derived via proteolytic digestion of intact antibodies (See, *e.g.*, Morimoto et al., Journal of Biochemical und Biophysical Methods 24: 107-1 17 (1992) and Brennan et al., Science, 229: 81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')₂ fragments (Carter et al., Biotechnology 10: 163- 167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent NO. 5,571,894; and US Patent No. 5,587,458. The antibody fragment may also be a "linear antibody", e.g., as described in US Patent 5,641,870 for example. Such linear antibody fragments may be monospecific or bispecific.

The term **"diabodies"** refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 0 404 097; WO 93/11161; and Hollinger et al., Proc. Natl. Acad. Sci. USA, 905444 - 6448 (1993).

The term **"antibody"** herein is used in the broadest sense and specifically covers intact monoclonal antibodies, polyclonal antibodies, multispecific antibodies (*e.g*. bispecific antibodies) formed from at least two intact antibodies, and antibody fragments so long as they exhibit the desired biological activity.

**"Native antibodies"** are usually heterotetrameric glycoproteins of about 150,000 Daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term **"variable"** refers to the fact that certain Portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (See Kabat et al., Sequences of Proteins Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

**"Fv" is the minimum antibody fragment** which contains a complete antigen-recognition and antigen-binding site. This region consists of one heavy chain and one light chain variable domain in tight, non-covalent association. It is in this configuration that the three hypervariable regions of each variable domain interact to define an antigen-binding site on the surface of the V_{H}-V_{L}. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab'), antibody fragments originally were produced as pairs of Fab'₂-fragments which have hinge cysteines between them. Other chemical couplings of antibody fragments are also known.

The variable domains comprises the 3 CDRs (hypervariable domains) and 4 framework domains (FR), flanking the CDRs. For example such a sequence will have the domains: FR - CRD1 - FR' - CDR2 - FR" - CDR3 - FR"'.

The **"light chains" of antibodies** (immunoglobulins) from any vertebrate species can be assigned to one of two clearly distinct types, called kappa (κ) and lambda (λ), based on the amino acid sequences of their constant domains.

Depending on the amino acid sequence of the constant domain of their **heavy chains,** antibodies can be assigned to different classes. There are five major classes of intact antibodies: IgA, IgD, IgE, IgG, and IgM, and several of these may be further divided into subclasses (isotypes), *e.g*., IgG1, IgG2, IgG3, IgG4, IgA, and IgA2. The heavy-chain constant domains that correspond to the different classes of antibodies are called α, δ, ε, γ, and µ respectively. The subunit structures and three-dimensional configurations of different classes of immunoglobulins are well known.

**" Single-chainFv" or "scFv "** antibody fragments comprise the V_{H} and V_{L} domains of antibody, wherein these domains are present in a Single Polypeptide chain. Preferably, the Fv Polypeptide further comprises a Polypeptide linker between the V_{H} and V_{L}, domains which enables the scFv to form the desired structure for antigen binding. For a review of scFv See Plückthun in The Pharmacology of Monoclonal Antibodies, vol. 113, Rosenburg and Moore eds., Springer-Verlag, New York, pp. 269-315 (1994).

In a further embodiment, antibodies or **antibody fragments can be isolated from antibody phage libraries** generated using the techniques described in McCafferty et al., Nature, 348: 552-554 (1990). Clackson et al., Nature, 352: 624-628 (1991) and Marks et al., J. Mol. Biol., 222: 581-597 (1991) describe the isolation of murine and human antibodies, respectively, using phage libraries. Subsequent publications describe the production of high affinity (mM range) human antibodies by chain shuffling (Marks et al., BiolTechnology, 10: 779-783 (1992)), as well as combinatorial infection and *in vivo* recombination as a strategy for constructing very large phage libraries (Waterhouse et al., Nuc. Acids. Res., 21: 2265-2266 (1993)). Libraries of synthetic genes based on the human antibody gene repertoire have also been successfully used for the isolation of therapeutic and diagnostic antibodies (HuCAL library; Knappik et al., J Mol Biol 296(1):57-86 (2000). Thus, these techniques are viable alternatives to traditional monoclonal antibody hybridoma techniques for isolation of monoclonal antibodies. The DNA also may be modified, for example, by substituting the coding sequence for human heavy- and light-chain constant domains in place of the homologous murine sequences (U.S. Patent No. 4,816,567; Morrison, et al., Proc. Nat. Acad. Sci. USA, 8 1: 685 1 (1984)), or by covalently joining to the immunoglobulin coding sequence all or part of the coding sequence for a non-immunoglobulin polypeptide. Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

The term **"monoclonal antibody"** as used herein refers to an antibody obtained from a population of substantially homogeneous antibodies, *i. e*., the individual antibodies comprising the population are identical except for possible naturally occurring mutations that may be present in minor amounts. Monoclonal antibodies are highly specific, being directed against a Single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as sequiring production of the antibody by any particular method. For example, the monoclonal antibodies to be used in accordance with the present invention may be made by the hybridoma method first described by Kohler et al., Nature, 256: 495 (1975), or may be made by recombinant DNA methods (See, *e.g*., U.S. Patent No. 4,816,567).

The **"monoclonal antibodies" may also be isolated from phage antibody libraries** using the techniques described in Clackson et al., Nature, 352: 624-628 (1991) and Marks et al., J. Mol. Biol., 222: 581-597 (1991), for example.

The monoclonal antibodies herein specifically include **"chimeric" antibodies** (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81: 685 1-6855 (1984)).

Chimeric antibodies of interest herein include "primatised" antibodies comprising variable domain antigen-binding sequences derived from a non-human primate *(e.g.* Old World Monkey, such as baboon, rhesus or cynomolgus monkey) and human constant region sequences (US Pat No. 5,693,780).

**Humanised antibodies:** Methods for humanising non-human antibodies have been described in the art. Preferably, a humanised antibody has one or more amino acid residues introduced into it from a source which is non-human. These non-human amino acid residues are often referred to as "import" residues, which are typicality taken from an "import" variable domain. Humanisation can be essentially performed following the method of Winter and Co-workers (Jones et al., Nature, 321: 522-525 (1986); Riech-mann et al., Nature, 332: 323-327 (1988); Verhoeyen et al., Science, 239: 1534-1536 (1988)), by substituting hypervariable region sequences for the corresponding sequences of a human antibody. Accordingly, such "humanised" antibodies are chimeric antibodies (U.S. Patent No. 4,816,567) wherein substantially less than an intact human variable domain has been substituted by the corresponding sequence from a non-human species. In practice, humanised antibodies are typically human antibodies in which some hypervariable region residues and possibly some FR residues are substituted by residues from analogous sites in rodent antibodies. The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanised antibody (Sims et al., J. Immunol., 151: 2296 (1993); Chothia et al., J. Mol. Biol., 196: 901 (1987)). Another method uses a particular framework region derived from the consensus sequence of all human antibodies of a particular subgroup of light or heavy chains. The same framework may be used for several different humanised antibodies (Carter et al., Proc. Natl. Acad. Sci. USA, 89: 4285 (1992); Presta et al., J. Immunol., 151: 2623 (1993)). It is further important that antibodies be humanized with retention of high affinity for the antigen and other favourable biological properties. To achieve this goal, according to a preferred method, humanised antibodies are prepared by a process of analysis of the parental sequences and various conceptual humanised products using three - dimensional models of the parental and humanized sequences. Three - dimensional immunoglobulin models are commonly available and are familiar to those skilled in the art. Computer programs are available which illustrate and display probable three-dimensional conformational structures of selected candidate immunoglobulin sequences.

Inspection of these displays permits analysis of the likely role of the residues in the functioning of the candidate immunoglobulin sequence, *i. e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

**Human antibodies:** As an alternative to humanisation, human antibodies can be generated. For example, it is now possible to produce transgenic animals *(e.g*., mice) that are capable, upon immunisation, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g*., Jakobovits et al., Proc. Natl. Acad. Sci. USA, 90: 2551 (1993); Jakobovits et al., Nature, 362: 255-258 (1993); Bruggermann et al., Year in Immuno., 7: 33 (1993); and US Patent Nos. 5,591,669;5,589,369 and 5,545,807.

Alternatively, **phage display technology** (McCafferty et al., Nature 348: 552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson, Kevin S. and Chiswell, David J., Current Opinion in Structural Biology 3: 564 - 571(1993). Several sources of V-gene Segments can be used for phage display. Clackson et al., Nature, 352: 624 - 628 (1991) isolated a diverse array of anti-oxazolone antibodies from a small random combinatorial library of V genes derived from the Spleens of immunized mice. A repertoire of V genes from unimmunized human donors can be constructed and antibodies to a diverse array of antigens (including self-antigens) can be isolated essentially following the techniques described by Marks et al., J. Mol. Biol. 222581 - 597 (1991), or Griffith et al., EMBO J. 12: 725 - 734 (1993). See, also, US Patent Nos. 5,565,332 and 5,573,905. Human antibodies may also be generated by *in vitro* activated B cells (see US Patents 5,567,610 and 5,229,275).

The term **"hypervariable region"** when used herein refers to the amino acid residues of an antibody which are responsible for antigen-binding. The hypervariable region comprises amino acid residues from a "complementarily determining region" or "CDR" *(e.g*. residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" *(e.g.* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196: 901-917 (1987)). "Framework" or "FR" residues are those variable domain residues other than the hypervariable region residues as herein defined.

**Amino acid sequence modification(s) of protein or antibody - fragment** described herein are contemplated. For example, it may be desirable to improve the binding affinity and/or other biological properties of the antibody part. Amino acid sequence variants of the antibody part are prepared by introducing appropriate nucleotide changes into the antibody part nucleic acid, or by peptide synthesis. Such modifications include, for example, deletions from, and/or insertions into and/or Substitutions of, residues within the amino acid sequences of the antibody part. Any combination of deletion, insertion and substitution is made to arrive at the final construct, provided that the final construct possesses the desired characteristics. Therefore, in the case of the heavy or light chain a variation of 1; 2; 3; 4; 5; 6; 7; 8; 9; 10; 11; 12; 13; 14; 15; 16; 17; or 20 amino acids can be executed. In the case of a linker a variation of 1; 2; 3; 4; 5; 6; or 7 can be executed. In case of the linker the variations are much more flexible, because function is simple to create a sufficient space between the functional amino acid sequences. A variation is defined as a deletion, insertion and/or substitution. The amino acid changes also may alter post-translational processes of the antibody part, such as changing the number or position of glycosylation sites. A useful method for identification of certain residues or regions of the antibody part that are preferred locations for mutagenesis is called "alanine scanning mutagenesis" as described by Cunningham and Welk Science, 244: 1081-1085 (1989). Here, a residue or group of target residues are identified *(e.g*., charged residues such as Arg, Asp, His, Lys, and Glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the Performance of a mutation at a given site, Ala scanning or random mutagenesis is conducted at the target codon or region and the expressed antibody part variants are screened for the desired activity. Amino acid sequence insertions include amino- and/or carboxyl-terminal fusions ranging in length from one residue to Polypeptides containing a hundred or more residues, as well as intrasequence insertions of single or multiple amino acid residues. Examples of terminal insertions include an antibody part with an N-terminal methionyl residue or the antibody part fused to a cytotoxic polypeptide. Other insertional variants of the antibody part molecule include the fusion to the N- or C-terminus of the antibody part of an enzyme, or a polypeptide which increases the serum half-life of the antibody part. Another type of variant is an amino acid substitution variant. These variants have at least one amino acid residue in the antibody part replaced by different residue. The sites of greatest interest for substitutional mutagenesis of antibody parts include the hypervariable regions, but FR alterations are also contemplated. Conservative substitutions are shown in Table 1 under the heading of "preferred substitutions".

If such substitutions result in a change in biological activity, then more substantial changes, denominated "exemplary substitutions" in Table 1, or as further described below in reference to amino acid classes, may be introduced and the products screened.

**Table 1**

| **Original Residue** | **Exemplary substitution** | **Preferred Substitution** |
|---|---|---|
| Ala | Val; Leu; Ile | Val |
| Arg | Lys, Gln, Asn | Lys |
| Asn | Gln, His, Asp, Lys, Arg | Gln |
| Asp | Glu, Asn | Glu |
| Cys | Ser, Ala | Ser |
| Gln | Asn, Glu | Asn |
| Glu | Asp, Gln, | Asp |
| Gly | Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, Gln, Asn | Arg |
| Met | Leu, Phe, Ile, | Leu |
| Phe | Leu, Val, Ile, Ala, Tyr | Tyr |
| Pro | Ala | Ala |
| Ser | Thr | Thr |
| Thr | Ser | Ser |
| Trp | Tyr, Phe | Tyr |
| Tyr | Trp, Phe, Thr, Ser | Phe |
| Val | Ile, Leu, Met, Phe, Ala, Norleucine | Leu |

Substantial modifications in the biological properties of the antibody part are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: Norleucine, Met, Ala, Val, Leu, Ile;
(2) neutral hydrophilic: Cys, Ser, Thr;
(3) acidic: Asp, Glu;
(4) basic: Asn, Gln, His, Lys, Arg;
(5) residues that influence chain orientation: Gly, Pro; and
(6) aromatic: Trp, Tyr, Phe.

Non-conservative substitutions will entail exchanging a member of one of these classes for another class. Any cysteine residue not involved in maintaining the proper conformation of the antibody part also may be substituted, generally with serine, to improve the oxidative stability of the molecule and prevent aberrant cross linking. Conversely, cysteine bond(s) may be added to the antibody part to improve its stability.

A particularly preferred type of substitutional variant involves substituting one or more hypervariable region residues of a parent antibody. Generally, the resulting variant(s) selected for further development will have improved biological properties relative to the parent antibody from which they are generated. A convenient way for generating such substitutional variants is affinity maturation using phage display. Briefly, several hypervariable region sites (*e.g.* 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity *(e.g.* binding affinity) as herein disclosed. In order to identify candidate hypervariable region sites for modification, alanine scanning mutagenesis can be performed to identify hypervariable region residues contributing significantly to antigen binding. Alternatively, or in additionally, it may be beneficial to analyse a crystal structure of the antigen-antibody complex to identify contact points between the antibody and antigen. Such contact residues and neighbouring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody part alters the original glycosylation pattern of the antibody part. By altering is meant deleting one or more carbohydrate moieties found in the antibody part, and/or adding one or more glycosylation sites that are not present in the antibody part.

Glycosylation of polypeptides, here the antibody parts, is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody part is conveniently accomplished by altering the amino acid sequence such that it contains one or more of the above-described tri - peptide sequences (for N-linked glycosylation sites). The alteration may also be made by the addition of, or substitution by, one or more serine or threonine residues to the sequence of the original antibody part (for O-linked glycosylation sites).

Nucleic acid molecules encoding amino acid sequence variants of the antibody part are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody part.

It may be desirable to modify the antibody part of the invention with respect to effector function, *e.g.* so as to enhance antigen-dependent cell-mediated cyotoxicity (ADCC) and/or complement dependent cytotoxicity (CDC) of the antibody part. This may be achieved by introducing one or more amino acid substitutions in an Fc region of an antibody part. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumour activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989).

Variations of the antibody fragments are defined by variations as mentioned before. That means the variations of special sequences, mentioned in the following list of Table 2, which can be modified be deletion, insertion and/or substitution by the following numbers of amino acids:

**Table 2**

| Seq. Id. No. | Numbers of amino acids, which are deleted, inserted and / or substituted: |
|---|---|
| 1, 4, 5, 6, 7, 10, 11, 12, 13, 16, 17, 18 | 1, 2 ,3, 4 |
| 2, 3, 8, 9, 14, 15 | 1,2 3, 4, 5, 6, 7, 8 |
| 19, 20, 21, 22, 23, 24 | 1, 2 , 3, 4, 5, 6, 7, 8, 9, 10, 11, 12, 13, 14,15, 16 17, 18, 19, 20, 21, 22 ,23 ,24, 25, 26, 27, 28, 29, 30 |
| 25, 26, 27, 28, 29, 30, | 1, 2 ,3, 4, 5, 6, 7, 8, 9, 10, 11, 12, 13, 14, 15, 16, 17, 18, 19, 20, 21, 22 ,23 ,24, 25, 26, 27, 28, 29, 30, 31, 32, 33, 34, 35, 36, 37, 38, 39, 40, 41, 42, 43, 44, 45, 46, 47, 48, 49, 50 |

### Use as a medicament

The antibody fragments of the invention exhibit pharmacological activity and are, therefore, useful as pharmaceuticals. In particular, the antibody fragments show pharmacological activity in a number of pathological or disease states in connection with a cancer, especially solid cancer, more especially breast cancer.

The antibody fragments of the invention may also be employed, alone or in combination (simultaneously or in doses at different times).

For the treatment of such conditions, the appropriated dosage will, of course, vary depending upon, for example, the host, the mode of administration and the nature and severity of the condition being treated.

Antibody fragments of the present invention may be administered to a patient in need of treatment via any suitable route, usually by invention into the bloodstream and/or directly into the site to be treated, e.g. tumour. The precise dose will depend upon a number of factors, the route of treatment, the size and location of the area to be treated (e.g. tumour), the precise nature of the antibody fragment (e.g. Fab', F(ab')2 , scFv molecule), and the nature of any detectable label or other molecule attached to the antibody. A typical antibody fragment dose will be in the range 5-50 mg/kg. This is a dose for a single treatment of an adult patient, which may be proportionally adjusted for children and infants, and also adjusted for other antibody formats in proportion to molecular weight. Treatments may be repeated at daily, twice-weekly, weekly or monthly intervals, at the discretion of the physician.

The present invention also provides pharmaceutical compositions comprising the antibody fragment of the invention (specifically recognising the EphB4 receptor) in association with at least one pharmaceutically acceptable carrier or diluent. Such composition may be manufactured in conventional manner. Pharmaceutically acceptable carriers or diluents are described in Remington's Pharmaceutical Science, 15th ed. Mack Publishing Company, Easton Pennsylvania (1980).

The treatment comprises therapeutical treatment.

The invention discloses further
(α) the use of the pharmaceutical composition of the antibody fragments of the invention for manufacture of a medicament for treatment of cancer especially breast cancer;
(β) a method of treatment of cancer especially breast cancer; which method comprises an administration of a pharmaceutical composition of the antibody fragments of the invention, wherein the amount of combination suppresses the disease and wherein the pharmaceutical composition of the combination is given to a patient, who is in need thereof;
(γ) a pharmaceutical composition of the combination of the invention for treatment of cancer especially breast cancer, which treatment comprises a pharmaceutical composition of the invention and at least one pharmaceutically acceptable carrier or diluent.
The preferred method of administration is the injection of the antibody fragment, the parenteral administration.

Preferred antibody fragments of the invention have an affinity (K_{D}) of at least 100 nM, more preferably of at least 10 nM human and/or murine EphB4 and can bind both native and recombinant EphB4. The K_{D} of antibody fragment binding to EphB4 can be assayed using any method known in the art, including technologies such as real-time Bimolecular Interaction Analysis Interaction Analysis (BIA) (Sjolander & Urbaniczky, Anal. Chem. 63, 2338-45, 1991, and Szabo et al., Curr. Opin. Struct. Biol. 5, 699-705, 1995). BIA can be used to study biospecific interactions in real time without labelling any of the interactants (e.g., BIACORE^{™}). Changes in the optical phenomenon surface plasmon resonance (SPR) can be used as an indication of real-time reactions between biological molecules.

In a BIACORE^{™} assay, preferred antibody fragments of the invention specifically bind to human EphB4 with a K_{D} of about 1 nM to about 40 nM. More preferred human antibodies specifically bind to human EphB4 with a K_{D} of equal or less than 1, 1.1, 1.3, 1.8, 2, 3, 4, 5, 6, 7, 8, 10, 12, 16, 20, 25, 30, 30, 35, or 40 nM.

A human antibody can be produced using chemical methods to synthesize its amino acid sequence, such as by direct peptide synthesis using solid-phase techniques (Merri-field, J. Am. Chem. Soc. 85, 2149-54, 1963; Roberge et al., Science 269, 202-04, 1995). Protein synthesis can be performed using manual techniques or by automation. Automated synthesis can be achieved, for example, using Applied Biosystems 431A Peptide Synthesizer (Perkin Elmer). Optionally, fragments of a human antibody can be separately synthesized and combined using chemical methods to produce a full-length molecule.

The newly synthesized molecules can be substantially purified by preparative high performance liquid chromatography (*e.g*., Creighton, PROTEINS: STRUCTURES AND MOLECULAR PRINCIPLES, W H Freeman and Co., New York, N.Y., 1983). The composition of a synthetic polypeptide can be confirmed by amino acid analysis or sequencing (e.g., using Edman degradation).

Preferably, however, antibodies of the invention are produced recombinantly by a host cell which has been transfected with a human antibody-encoding expression construct, as described below. The host cell is cultured in conditions under which the antibodies are expressed. Antibodies can be purified from the host cell and can be separated from other compounds that normally associate with the antibodies in the cell (such as proteins, carbohydrates, or lipids) using methods well known in the art. Such methods include, but are not limited to, size exclusion chromatography, ammonium sulphate fractionation, ion exchange chromatography, affinity chromatography, and preparative gel electrophoresis. Alternatively, antibodies can be produced containing a signal sequence which causes their secretion into the culture medium.

A preparation of purified antibody fragments of the invention is at least 80% pure (80% antibody, 20% other proteins), preferably, the preparations are 90%, 95%, or 99% pure. Purity of the preparations can be assessed by any means known in the art, such as SDS-polyacrylamide gel electrophoresis. A preparation of purified antibody fragments of the invention can contain more than one type of EphB4 antibody fragment.

The invention provides nucleic acid molecules, e.g. DNA molecules encoding all or a portion of EphB4 antibodies of the invention. The nucleic acid molecules may encode a light chain and/or a heavy chain. The nucleic acid can be used, for example, to produce quantities of the antibodies for therapeutic or diagnostic use.

A nucleic acid molecule of the invention can be inserted into an expression vector which contains the necessary elements for the transcription and translation of the inserted coding sequence, thereby forming an expression construct. Methods that are well known to those skilled in the art can be used to make expression constructs containing sequences encoding antibodies of the invention or portions thereof. These methods include in vitro recombinant DNA techniques, synthetic techniques, and *in vivo* genetic recombination. Such techniques are described, for example, in Sambrook et al., MOLECULAR CLONING: A LABORATORY MANUAL, 2d ed., 1989, and in Ausubel et al., CURRENT PROTOCOLS IN MOLECULAR BIOLOGY, John Wiley & Sons, New York, N.Y., 1995.

A variety of expression vector/host systems can be utilized to contain and express a nucleic acid molecule encoding a human antibody of the invention. These include, but are not limited to, microorganisms, such as bacteria transformed with recombinant bacteriophage, plasmid, or cosmid DNA expression vectors or yeast transformed with yeast expression vectors. Insect cell systems infected with virus expression vectors (*e.g*., baculovirus), plant cell systems transformed which, *e.g*., cauliflower mosaic virus, tobacco mosaic virus, or with a bacterial expression vector (*e.g*., Ti or pBR322 plasmids), or animal cell systems also can be used.

Control elements or regulatory sequences present in an expression construct are those non-translated regions (*e.g*., enhancers, promoters, 5' and 3' untranslated regions) which interact with host cellular proteins to carry out transcription and translation. Such elements can vary in their strength and specificity. Depending on the vector system and host used, any number of suitable transcription and translation elements, including constitutive and inducible promoters, can be used. For example, when cloning in bacterial systems, inducible promoters such as the hybrid lacZ promoter of the BLUESCRIPT® phagemid (Stratagene, LaJolla, Calif.), pSPORT1 plasmid (Life Technologies), and the like can be used. The baculovirus polyhedrin promoter can be used in insect cells. Promoters or enhancers derived from the genomes of plant cells (e.g., heat shock, RUBISCO, and storage protein genes) or from plant viruses (*e.g*., viral promoters or leader sequences) can be included in an expression construct. In mammalian cell systems, promoters from mammalian genes or from mammalian viruses are preferable. To generate a cell line which contains multiple copies of a nucleic acid molecule of the invention, constructs based an SV40 or EBV can be used with an appropriate selectable marker.

Large scale production of EphB4 antibody fragments can be carried out using methods such as those described in Wurm et al., Ann. N. Y. Acad. Sci. 782, 70-78, 1996, and Kim et al., Biotechnol. Bioengineer 58, 73-84, 1998.

The invention also provides methods of using EphB4 antibody fragments to modulate, e.g. to inhibit or prevent the binding of Ephrin B2 to EphB4. Such methods can be used therapeutically, as described below, or in a research setting, for example in drug screening.

Further, the invention provides therapeutic methods of inhibiting angiogenesis and treating angiogenesis-associated diseases or disorders including, but not limited to, angiogenesis-dependent cancers (*e.g*., solid tumours, leukemias, tumour metastases, benign tumours such as hemangiomas, acoustic neuromas, neurofibromas, trachomas, and pyogenic granulomas), inflammatory disorders (*e.g*., immune and non-immune inflammation), chronic articular rheumatism and psoriasis, ocular angiogenic diseases, Osler-Webber Syndrome, myocardial angiogenesis, plaque neovascularization, telangiectasia, hemophiliac joints, angiofibroma, and wounds.

The methods involve administering a therapeutic amount of a pharmaceutical composition of the invention (described below) to an individual who needs such therapy. Pharmaceutical compositions of the invention can be administered by any number of routes including, but not limited to, oral, intravenous, intramuscular, intra-arterial, intramedullary, intrathecal, intraventricular, transdermal, subcutaneous, intraperitoneal, intranasal, parenteral, topical, sublingual, or rectal means. Pharmaceutical compositions of the invention can be administered to a patient alone, or in combination with further medicaments (in the same or in a different composition), e.g. with further anti tumour-agents such as chemotherapeutic agents.

Pharmaceutical compositions of the invention comprise an EphB4 antibody fragment or a nucleic acid molecule of the invention and a pharmaceutically acceptable carrier. The pharmaceutically acceptable carrier preferably is non-pyrogenic. The compositions can be administered alone or in combination with at least one other agent, such as stabilizing compound, which can be administered in any sterile, biocompatible pharmaceutical carrier, including, but not limited to, saline, buffered saline, dextrose, and water. A variety of aqueous carriers may be employed, *e.g*., 0.4% saline, 0.3% glycine, and the like. These solutions are sterile and generally free of particulate matter. These solutions may be sterilized by conventional, well known sterilization techniques (*e.g*., filtration). The compositions may contain pharmaceutically acceptable auxiliary substances as required to approximate physiological conditions such as pH adjusting and buffering agents, etc.

The active agent in pharmaceutical compositions is an EphB4 antibody fragment. A given effective amount will vary from condition to condition and in certain instances may vary with the severity of the condition being treated and the patient's susceptibility to treatment. See U.S. Pat. No. 5,851,525. If desired, a pharmaceutical composition can comprise more than one type of antibody, for example with different K_{D} for EphB4 binding or with different IC₅₀s for inhibiting an EphB4 function, can be included in a pharmaceutical composition.

After pharmaceutical compositions have been prepared, they can be placed in an appropriate container and labelled for treatment of an indicated condition. Such Labelling would include amount, frequency, and method of administration.

A "therapeutically effective dose" is an amount of antibody fragment which reduces the severity of at least one symptom of the disorder being treated relative to the severity of the symptom which occurs in the absence of the therapeutically effective dose. Determination of a therapeutically effective dose is well within the capability of those skilled in the art. For example, a therapeutically effective dose can be estimated initially either in cell culture assays or in animal models, usually rats, mice, rabbits, dogs, or pigs. An animal model also can be used to determine the appropriate concentration range and route of administration. Such information can then be used to determine useful doses and routes for administration in humans.

Therapeutic efficacy and toxicity, *e.g.*, ED₅₀ (the dose therapeutically effective in 50% of the population) and LD₅₀ (the dose lethal to 50% of the population) of a human antibody, can be determined by standard pharmaceutical procedures in cell cultures or experimental animals. The dose ratio of toxic to therapeutic effects is the therapeutic index, and it can be expressed as the ratio LD₅₀/ED₅₀.

Pharmaceutical compositions which exhibit large therapeutic indices are preferred. The dosage contained in pharmaceutical compositions of the invention preferably is within a range of circulating concentrations which include the ED₅₀ with little or no toxicity. The dosage varies within this range depending upon the dosage form employed, sensitivity of the patient, and the route of administration.

The exact dosage will be determined by the practitioner, in light of factors related to the patient who requires treatment. Dosage and administration are adjusted to provide sufficient levels of the human antibody or to maintain the desired effect. Factors which can be taken into account include the severity of the disease state; general health of the subject; age, weight, and gender of the subject; diet; time and frequency of administration; drug combination(s); reaction sensitivities; and tolerance or response to therapy. Long-acting pharmaceutical compositions can be administered every 3 to 4 days, every week, or once every two weeks depending an the half-life and clearance rate of the particular formulation.

Effective *in vivo* dosages of a human antibody are in the range of about 5 mg to about 50 mg/kg, about 100 mg to about 500 mg/kg of patient body weight, and about 200 to about 250 mg/kg of patient body weight. For administration of nucleic acid molecules encoding the antibodies, effective *in vivo* dosages are in the range of about 100 ng to about 200 ng, 500 ng to about 50 mg, about 1 mg to about 2 mg, about 5 mg to about 500 mg, and about 20 mg to about 100 mg of DNA.

Those skilled in the art will understand that various changes in form and details may be made to the invention defined by the following examples and the appended claims. Such equivalents are encompassed within the scope of the claims.
FIG. 1: Graph showing specificity of anti-EphB4 Fab fragments.
FIG. 2: Graph showing inhibition of ligand-induced phosphorylation of EphB4.
FIG. 3: Graph showing EphB4 phosphorylation by dimeric anti-EphB4 Fabs.
FIG. 4: FACS analysis of MCF-7 breast cancer cell line with anti-EphB4 Fabs. Top panel A: unstained cells, middle panel B: stained with anti-EphB4 Fabs and secondary antibody (anti-lyso = anti-lysozyme negative control Fab), bottom panel C: secondary antibody only.
FIG. 5: Anti-EphB4 Fab precipitate human EphB4 receptor from CHO-EphB4 cells
FIG. 6: Sequence of the anti EphB4 Fab fragments MOR03640; MOR03639 and MOR03641 each heavy and light chain. The CDRs are written in bold, the constant parts are underlined, the purification part (poly His) are following the constant part of MOR03640_MH heavy chain and are not underlined.
FIG. 7: Specificity of Fabs for EphB4 and cross-reactivity to both human and murine EphB4 in ELISA
FIG. 8: Inhibition of Ephrin B2 binding to EphB4
FIG. 9: Anti-EphB4 Fab MOR 03640 binds to hEphB4-transfected CHO cells
FIG. 10: Inhibition of mEphrin B2 binding to mEphB4
FIG. 11: Inhibition of mEphrin B binding to hEphB4
FIG. 12: Inhibition of mEphrin B2 binding to CHO-hEphB4 cells

### EXAMPLES

### EXAMPLE 1

### Isolation of EphB4-specific Fab antibody fragments

Synthetic Fab antibody fragments were isolated from the HuCAL GOLD® antibody library (HuCAL®; Knappik et al., J. Mol. Biol 296, 57-86, 2000). Selection strategy and screening on both recombinant antigens and transfected cells ensured isolation and identification of Fabs cross-reactive to both the murine and the human EphB4 extracellular domain. Three Fab antibody fragments were identified. The Fab fragments comprise VH-CH1 and VL-CL nucleic acids.

Specificity for EphB4 in ELISA: Wells of a 96-well maxisorp-plate were coated overnight with 100µL recombinant human EphB4 (hEphB4)-, recombinant mouse EphB3 (mEphB3)- or recombinant mouse EphA4 (mEphA4)-Fc fusion protein at a final concentration of 2,5µg/mL in PBS. Anti-EphB4 Fab fragments (purified, MH-tagged) were added (100µL/well, 100µg/mL final concentration in chemiblock) to respective wells and incubated for 1h at RT. Then 100µl mEphrin B2-Fc fusion protein either spiked with the respective anti-EphB4 Fab or with PBS was added (25ng/ml mEphrinB2 +/- 100µg/ml Fab diluted in chemiblock) and incubated for max. 20min at RT. The amount of bound Ephrin B2 was detected using biotinylated goat anti-Ephrin B2 antibody (0,05µg/ml final concentration diluted in chemiblock, 1h at RT), AP-conjugated streptavidin and AttoPhos substrate. Fluorescence was measured (RFU) at 535nm (430nm excitation).

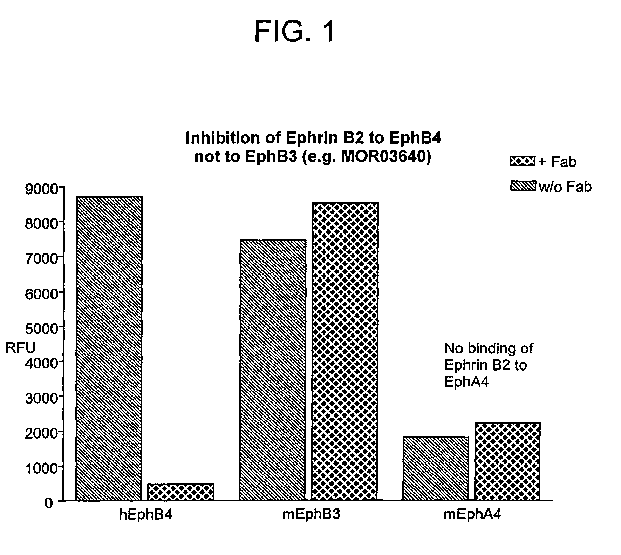

The results are shown in FIG. 1.
Binding of the ligand EphrinB2 to its receptor can potently be blocked by the anti-EphB4 Fab MOR03640: a high fluorescence signal (RFU) indicates unhindered ligand-receptor,binding as is the case for mEphrin B2 binding to mEphB3 . On the other hand binding of the ligand mEphrin B2 to the receptor hEphB4 is clearly prevented by addition of the anti-EphB4 Fab indicating that this Fab show specific inhibition of the mEphrin B2-hEphB4 interaction.

### EXAMPLE 2

### Affinity of anti-EphB4 Fabs

The affinity of the antibody fragments was determined using BIACORE^{™} analysis and cell ELISA. The Biacore chip was coated with recombinant human or murine EphB4-Fc fusion protein. The results are shown in Table 3.

**Table 3**

| Antibody | K_{D} (nM) for EphB4 | |
|---|---|---|
| | Human | Murine |
| MOR03639 | 13 ± 3 | 43 ± 4 |
| MOR03640 | 1.8 ± 0.7 | 4 ± 0.4 |
| MOR03641 | 35 ± 5 | 151 ± 21 |

The Fabs show nanomolar affinities for both the murine and the human EphB4 receptor (extracellular domains). MOR03640 shows the highest affinities for both receptors, with very similar affinities for the two different species.

IC₅₀ values were determined using recombinant antigen (murine and human EphB4) in an ELISA and on transfected cells in a FACS analysis (human EphB4 receptor). The results are shown in Table 4.

**Table 4.**

| Antibody | IC₅₀ (µg/mL) inhibition human EphB4 | | IC₅₀ (µg/mL) inhibition murine EphB4 |
|---|---|---|---|
| | ELISA | FACS | ELISA |
| MOR03639 | 65 ± 5 | 2.8 | 22 ± 8 |
| MOR03640 | 33 ± 9 | 2.1 | 117 ± 21 |
| MOR03641 | 5 ± 0.8 | 4,4 | 24 ± 0.2 |

Binding of (a fixed concentration of) the ligand Ephrin B2-Fc to immobilized EphB4 receptor on an ELISA plate or on transfected cells is titrated with anti-EphB4 Fab. The IC50 is determined as the amount of Fab at which the binding of Ephrin B2 is inhibited by 50%.

### EXAMPLE 3

### Inhibition of hEphB4 phosphorylation by monomeric Fab

CHO cells were transfected with human EphB4 containing a myc tag. Transfected cells were seeded into 96-well microtiter plates and incubated at 37°C, 5% CO₂ for 24 hours. Monomeric EphB4 Fabs were added to the wells in different concentrations and incubated for 30 minutes, then Ephrin B2-Fc was added to the wells in a final concentration of 250 ng/mL. After a further incubation for 30 minutes, the cells were washed once with PBS and then lysed. EphB4 in the lysates was captured via its myc-tag. Phosphorylation was detected with a peroxidase-coupled anti-phospho-tyrosine antibody.

The results are shown in FIG. 2. The ligand-induced phosphorylation (activation) of the EphB4 receptor on the transfected CHO cells is measured via a peroxidase-coupled anti-phospho-tyrosine antibody. Positive control: 100% phosphorylation of the receptor by Ephrin B2 alone, negative control: Ephrin B2 plus an irrelevant Fab antibody; this Fab does not bind to the EphB4 receptor, thus does not inhibit binding of the Ephrin B2 ligand to the receptor and therefore also leads to 100% phosphorylation of the receptor. The anti-EphB4 Fabs 3639, 3640 and 3541 block binding of the Ephrin B2 ligand to the EphB4 receptor, resulting in strongly reduced phosphorylation of the receptor.

### EXAMPLE 4

### Phosphorylation of EphB4 by dimeric Fab

The assay was performed as described above, with the following modifications: dimeric Fab fragments (20 µg/mL) were pre - incubated with an anti-myc tag antibody at 60 or 120 µg/mL at room temperature for 30 minutes before the mixture was added to the cells. As a positive control, one set of wells was incubated with Ephrin B2-Fc only at a concentration of 250ng/mL.

The results are shown in FIG. 3. Dimeric Fab antibody fragments mimic the Ephrin B2 ligand, leading to a strong phosphorylation of the EphB4 receptor.

### EXAMPLE 5

### MCF- 7 breast cancer cell line is EphB4 positive

FACS analysis was used to confirm that the MCF-7 breast cancer cell line is highly positive for EphB4 expression. EphB4 Fabs were incubated at a concentration of 10 µg/mL with 10⁶ MCF-7 cells at 4°C for 1 hour. After washing with PBS, the cells were incubated with FITC-labelled secondary antibody. After a final PBS wash, the cell-associated fluorescence was measured using a Becton Dickinson Calibur Fluorescence activated cell sorter.

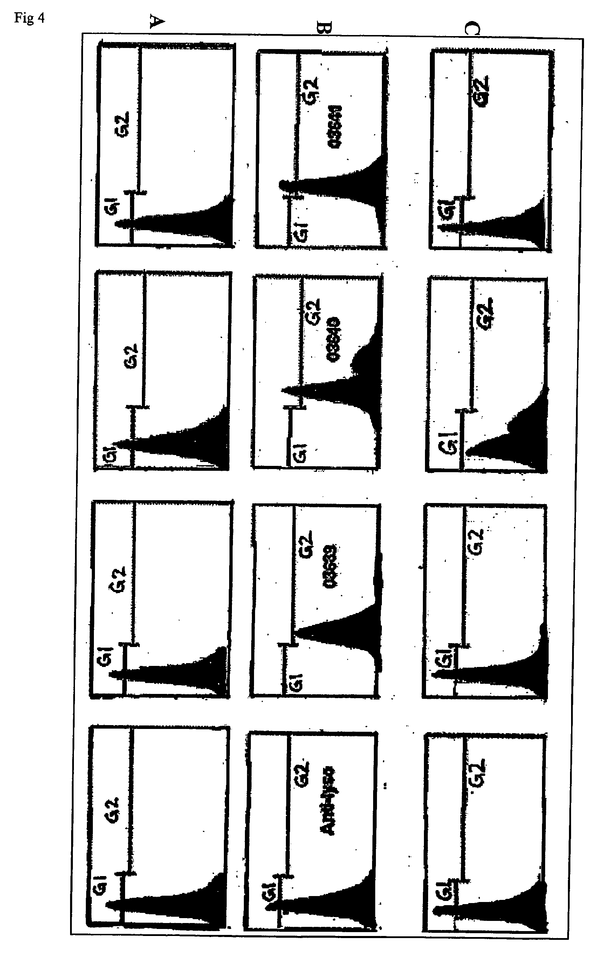

The results are shown in FIG. 4. Each column is the result of at least two independent experiments. The top panel shows unlabeled cells, the bottom panel cells incubated which the secondary antibody (anti-myc tag antibody) only. The middle panel Shows cells incubated with both first (negative control: anti-lysozyme Fab, anti-EphB4 Fabs 03639, 03640, 03641) and secondary antibodies. Upon incubation which the anti-EphB4 Fabs, the MCF-7 cells show a shift, indicating recognition of the cell-surface EphB4 receptor by the Fabs. The negative control Fab does not stain the cells.

### EXAMPLE 6

### Anti-EphB4 Fab precipitate human EphB4 receptor from CHO-EphB4 cells

### Method

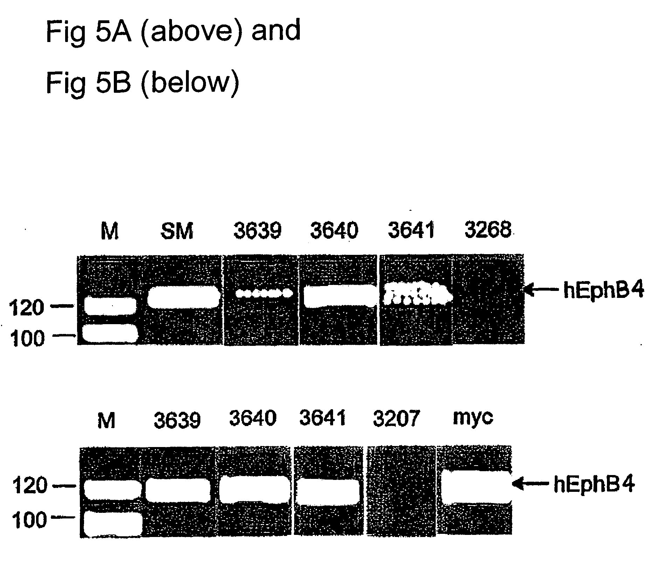

CHO-EphB4 cells (Sturz et al. 2003) were lysed in lysis buffer (50mM Hepes pH 7,2, 150mM NaCl, MgCl₂1 MM, 10% Glycerine(v/v), 1.5%Triton X-100 (v/v), 2% phosphatase inhibitor cocktail 2 (v/v, Sigma), 4% protease inhibitor cocktail complete (v/v, Roche) and the lysate was cleared by centrifugation and pre - absorbed to protein A agarose beads (4 fast flow; Sigma). For immunoprecipitation 5 µg of antibodies (dissolved to 0.5µg/µl in PBS) were added to 100µg (1 µg/µL) of cell lysate and incubated over night at 4°C. 10µg antibodies against human IgG (Jackson Immuno Research) pre - adsorbed to Protein A beads were added and incubated for 1 h at 4°C to collect the immunocomplexes. In the case of anti-myc antibody (mAb 9E10, Roche) only Protein A beads were added. Protein A beads were pelleted by centrifugation and washed for three times with 1 mL of lysis buffer and once with 1 mL of PBS. Bound material was removed with SDS gel loading buffer for 15 min at 70°C and separated an 4-12% SDS polyacrylamide gels in Tris/tricine buffer (Invitrogen). Proteins were transferred to nitrocellulose membranes and EphB4 was detected by Western blot using mouse monoclonal anti-myc antibodies and anti mouse IgG antibodies coupled with horseradish peroxidase (Amersham Pharmacia Biotech). The blot was developed using chemilumi-nescence. See FIG. 5A.

EphB4 was immunoprecipitated from CHO-EphB4 cell lysate as described in the methods section using monomeric EphB4 Fabs 3639, 3640 and 3641. An unrelated Fab was used as negative control (3268). M: Marker; SM: starting material. See FIG. 5B.

EphB4 was immunoprecipitated from CHO-EphB4 cell lysate as described in the methods section using dimeric EphB4 Fabs 3639, 3640 and 3641. An unrelated Fab was used as negative control (3207). As a positive control, the myc-tagged EphB4 was immunoprecipitated with a mAb against the myc epitope (myc). M: Marker.

### EXAMPLE 7

### Cross-reactivity of Fabs for human and murine EphB4 and specificity of Fabs for EphB4, not related Ephs

A 96-well plate was coated with recombinant mEphB1, -2, -3, -4, -6, mEphA4 and hEphB4 in PBS o/n at 4°C (5µg/mL; 100µL/well). The next day, the antigen was discarded and the plate blocked with 3% BSA/PBS (300µ/well) for 2 hrs at room temperature (RT). Blocking solution was discarded and anti-EphB4 Fabs were added to the wells at a concentration of 20µg/mL in 1% BSA/PBS and incubated for 1 hr at RT. Fabs were discarded and the plate was washed 3x with PBS. Anti-human F(ab')₂-HRP was added at a concentration recommended by the manufacturer in 1% BSA/PBS and incubated for 1 hr at RT. The plate was washed 3x with PBS and 100µL/well chemifluorescent substrate was added. After development, fluorescence was measured in a fluorescence reader.

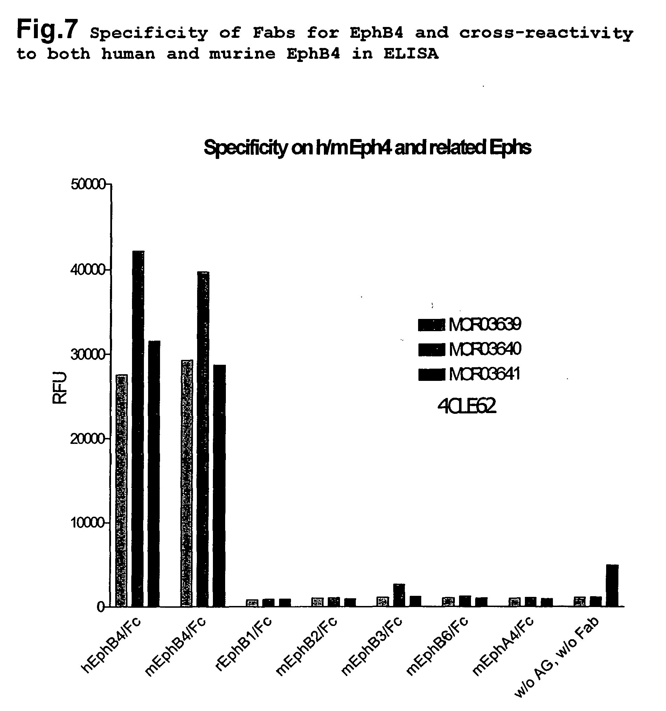

The results are shown in Fig. 7. The anti-EphB4 Fabs show specificity for EphB4 and are cross-reactive to both human and murine EphB4.

### EXAMPLE 8

### Inhibition of mEphrin B2 to hEphB4 not mEphB3

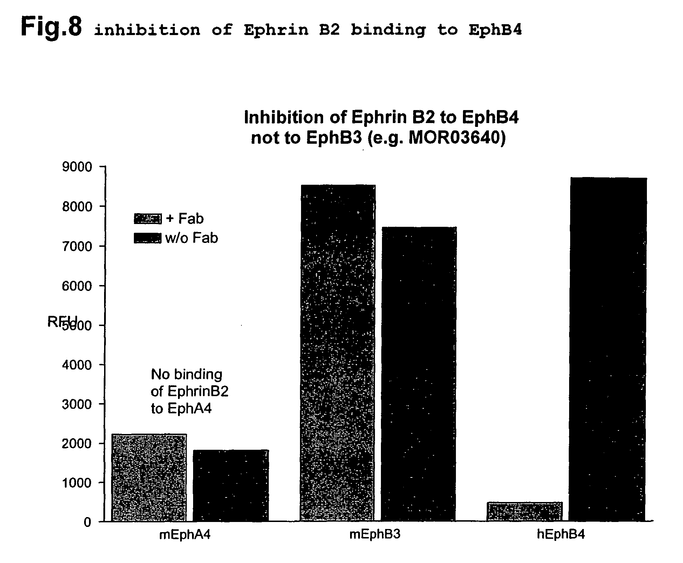

A 96-well plate was coated with recombinant mEphB3, mEphA4 and hEphB4 in PBS o/n at 4°C (5µ/mL; 100µL/well). The next day, the antigen was discarded and the plate blocked with 3% BSA/PBS (300µl/well) for 2 hrs at room temperature (RT). Blocking solution was discarded and anti-EphB4 Fabs were added to the wells at a concentration of 100µg/mL in 1% BSA/PBS and incubated for 30min at RT. Recombinant murine Ephrin B2 was added at a final concentration of 250ng/mL and incubated at RT for a further 30min. The plate was washed and bound Ephrin B2 was detected with Streptavidin-HRP (incubation 30min at RT) and chemifluorescent substrate.
Result: see Fig. 8: Inhibition of EphrinB2 binding to EphA4 (unspecific binding), EphB3 (specific interaction, but not inhibited by anti-EphB4 HuCAL Fabs) and EphB4 (specific interaction, inhibited by HuCAL Fab).

### EXAMPLE 9

### FACS binding to hEphB4 expressing CHO cells

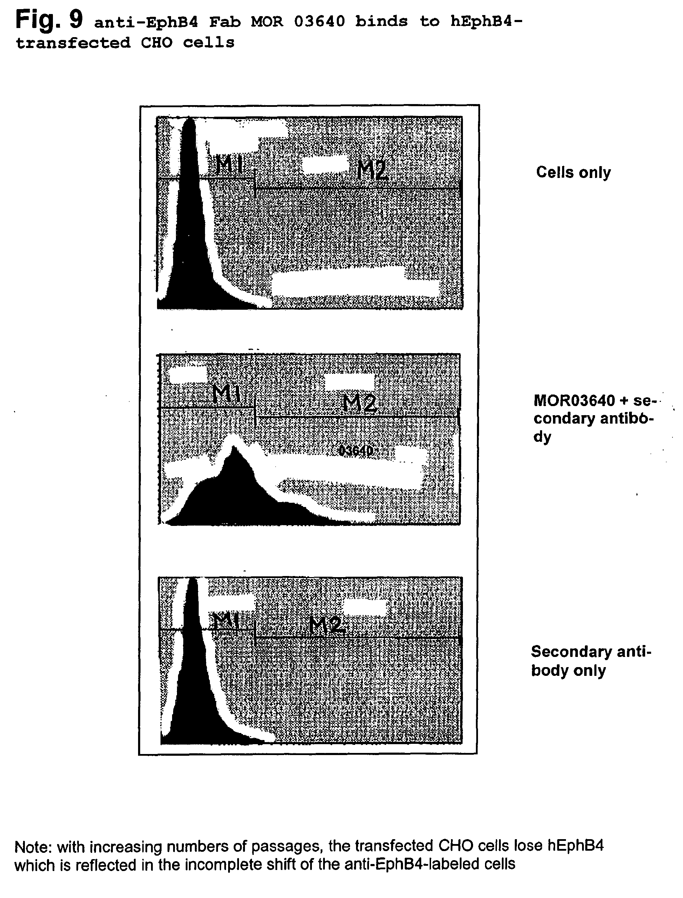

CHO cells transfected with a myc-tagged hEphB4 receptor (Sturz et al., 2004 Biochem Biophys Res Commun. 313(1):80-8) were trypsinized, washed and resuspended in FACS buffer (PBS/1%BSA) containing. anti-EphB4 antibody at a concentration of 10µg/mL and incubated at 4°C for 1hr. Cells were washed in FACS buffer and resuspended in FACS buffer containing goat anti-human IgG (FITC) diluted 1:100 (Jackson Immuno research, # 109-096-088) and incubated at 4°C for 1hr. After a final wash step, cell staining was analyzed in a Becton Dickinson FACS.
Result: see Fig. 9: anti-EphB4 Fab MOR03640 binds hEphB4-transfected CHO cells

### EXAMPLE 10

### Inhibition of mEphrin B2- mEphB4 interaction by anti-EphB4 Fab

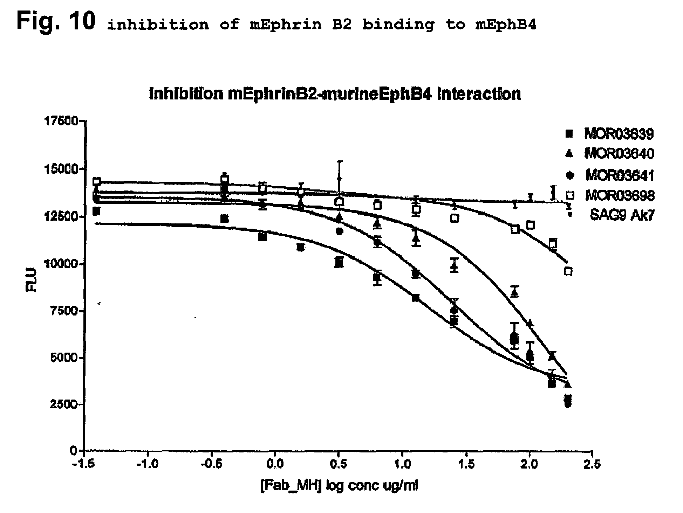

A 96-well plate was coated with recombinant mEphB4 in PBS o/n at 4°C (5µg/mL; 100µL/well). The next day, the antigen was discarded and the plate blocked with 3% BSA/PBS (300µl/well) for 2 hrs at room temperature (RT). Blocking solution was discarded and anti-EphB4 Fabs were added to the wells at different concentrations in 1% BSA/PBS and incubated for 30min at RT. Recombinant murine Ephrin B2 was added at a final concentration of 250ng/mL and incubated at RT for a further 30min. The plate was washed and bound Ephrin B2 was detected with Streptavidin-HRP (incubation 30min at RT) and chemifluorescent substrate.
Result: see Fig. 10: Inhibition of mEphrinB2 binding to mEphB4 by anti-EphB4 Fabs

### EXAMPLE 11

### Inhibition of mEphrin B2- hEphB4 interaction by anti-EphB4 Fab

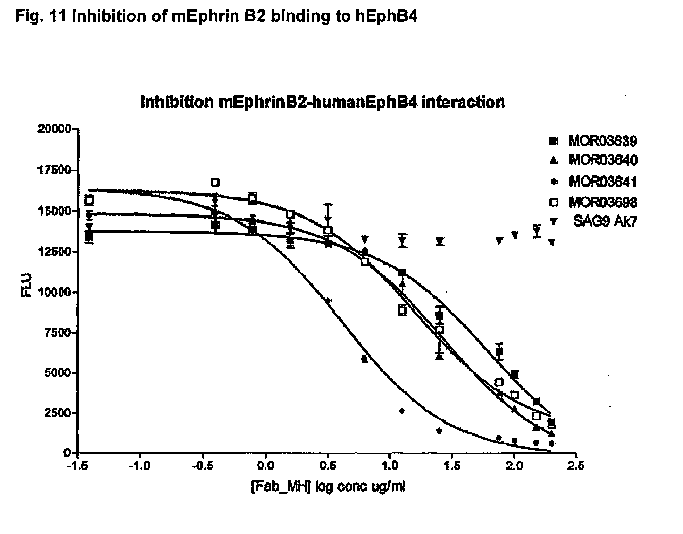

A 96-well plate was coated with recombinant hEphB4 in PBS o/n at 4°C (5µg/mL; 100µL/well). The next day, the antigen was discarded and the plate blocked with 3% BSA/PBS (300µl/well) for 2 hrs at room temperature (RT). Blocking solution was discarded and anti-EphB4 Fabs were added to the wells at different concentrations in 1% BSA/PBS and incubated for 30min at RT. Recombinant murine Ephrin B2 was added at a final concentration of 250ng/mL and incubated at RT for a further 30min. The plate was washed and bound Ephrin B2 was detected with Streptavidin-HRP (incubation 30min at RT) and chemifluorescent substrate.
Result: see Fig. 11: Inhibition of mEphrinB2 binding to hEphB4 by anti-EphB4 Fabs

### EXAMPLE 12

### Inhibition of mEphrin B2 binding to CHO-hEphB4 cells

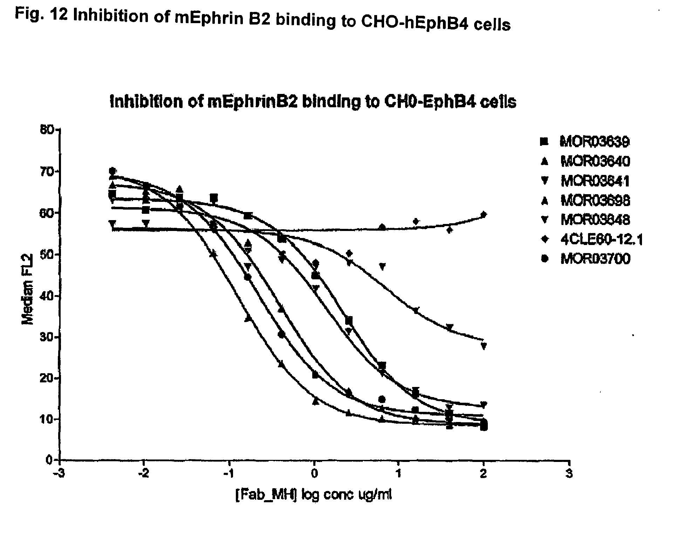

CHO-hEphB4 cells were trypsinized, washed and resuspended in FACS buffer (PBS/1%BSA) containing anti-EphB4 antibody at different concentrations and incubated at 4°C for 30min. Cells were washed in FACS buffer and resuspended in FACS buffer containing biotinylated mEphrin B2 at 250ng/mL and incubated at 4°C for 1 hr. cells were washed in FACS buffer and resuspended in FACS buffer containing Streptavidin-PE and incubated at 4°C for 30min. After a final wash step, cell staining was analyzed in a Becton Dickinson FACS.
Result: see Fig. 12: with increasing concentration, anti-EphB4 Fabs inhibit Ephrin B2 binding to hEphB4-transfected CHO cells

SEQUENCE IDENTIFIER

<110> Bayer Schering Pharma Aktiengesellschaft
<110> MorphoSys AG
<120> Anti EphB4 antibody parts
<130> 53475AEP
<140>
<141>
<160> 30
<170> PatentIn Ver. 2.1

<210> Sequence Identifier No.1
<211> 10
<212> PRT
<213> Artificial Sequence
<220>
<221> DOMAIN
<222> (1)...(10)
<223> 3640 CDR-H1

Gly Phe Thr Phe Asn Asn Ser Trp Met Ser
                                    10
<400> 1


<210> Sequence Identifier No.2
<211> 20
<212> PRT
<213> Artificial Sequence
<220>
<221> DOMAIN
<222> (1)...(20)
<223> 3640 CDR-H2

Trp Val Ser Asn Ile Thr Ser Ser Ser Ser Met Thr His Tyr Ala Asp
                                    10
Ser Val Lys Gly
            20
<400> 2


<210> Sequence Identifier No.3
<211> 17
<212> PRT
<213> Artificial Sequence
<220>
<221> DOMAIN
<222> (1)...(17)
<223> 3640 CDR-H3

Lys Gly Met Ser Phe Tyr Asn Asn Lys Tyr Ala Lys Ser Met Phe Asp
                                    10
Leu
<400> 3


<210> Sequence Identifier No.4
<211> 11
<212> PRT
<213> Artificial Sequence

<220>
<221> DOMAIN
<222> (1)...(11)
<223> 3640 CDR-L1

Ser Gly Asp Asn Leu Gly Lys Lys Tyr Val His
                                    10
<400> 4


<210> Sequence Identifier No.5
<211> 11
<212> PRT
<213> Artificial Sequence
<220>
<221> DOMAIN
<222> (1)...(11)
<223> 3640 CDR-L2

Leu Val Ile Tyr Tyr Asp Ser Lys Arg Pro Ser
                                    10
<400> 5


<210> Sequence Identifier No.6
<211> 10
<212> PRT
<213> Artificial Sequence
<220>
<221> DOMAIN
<222> (1)...(10)
<223> 3640 CDR-L3

Ser Ala Trp Thr Thr Thr Met Met His Ile
                                    10
<400> 6


<210> Sequence Identifier No.7
<211> 10
<212> PRT
<213> Artificial Sequence
<220>
<221> DOMAIN
<222> (1)...(10)
<223> 3639 CDR-H1

Gly Phe Thr Phe Ser Asn Tyr Tyr Leu His
                                    10
<400> 7


<210> Sequence Identifier No.8
<211> 17
<212> PRT
<213> Artificial Sequence
<220>
<221> DOMAIN
<222> (1)...(17)
<223> 3639 CDR-H2

Gly Ile Ser Tyr Ser Gly Ser Ser Thr Tyr Tyr Ala Asp Ser Val Lys
                                        10
Gly
<400> 8


<210> Sequence Identifier No.9
<211> 13
<212> PRT
<213> Artificial Sequence
<220>
<221> DOMAIN
<222> (1)...(13)
<223> 3639 CDR-H3

Phe Tyr Gln Arg Arg Gln Ile Pro His Gly Tyr Asp Val
                                        10
<400> 9


<210> Sequence Identifier No.10
<211> 11
<212> PRT
<213> Artificial Sequence
<220>
<221> DOMAIN
<222> (1)...(11)
<223> 3639 CDR-L1

Ser Gly Asp Asn Ile Gly Asp Tyr Tyr Val His
                                        10
<400> 10


<210> Sequence Identifier No.11
<211> 11
<212> PRT
<213> Artificial Sequence
<220>
<221> DOMAIN
<222> (1)...(11)
<223> 3639 CDR-L2

Leu Val Ile Tyr Arg Asp Ser Asn Arg Pro Ser
                                        10
<400> 11


<210> Sequence Identifier No.12
<211> 10
<212> PRT
<213> Artificial Sequence
<220>
<221> DOMAIN
<222> (1)...(10)
<223> 3639 CDR-L3

Gly Ser Tyr Asp Met Pro Phe Gly Gly Thr
                                        10
<400> 12

<210> Sequence Identifier No.13
<211> 10
<212> PRT
<213> Artificial Sequence
<220>
<221> DOMAIN
<222> (1)...(10)
<223> 3641 CDR-H1

Gly Gly Thr Phe Ser Thr Tyr Ala Ile Ser
                                    10
<400> 13


<210> Sequence Identifier No.14
<211> 20
<212> PRT
<213> Artificial Sequence
<220>
<221> DOMAIN
<222> (1)...(20)
<223> 3641 CDR-H2

Trp Met Gly Arg Ile Ile Pro Ile Phe Gly Thr Ala Asn Tyr Ala Gln
                                    10
Lys Phe Gln Gly
            20
<400> 14


<210> Sequence Identifier No.15
<211> 17
<212> PRT
<213> Artificial Sequence
<220>
<221> DOMAIN
<222> (1)...(17)
<223> 3641 CDR-H3

Ser Ser Tyr Thr Ile Leu Thr Thr Tyr Tyr Asn Tyr Asn Ala Met Asp
                                    10
Asn
<400> 15


<210> Sequence Identifier No.16
<211> 11
<212> PRT
<213> Artificial Sequence
<220>
<221> DOMAIN
<222> (1)...(11)
<223> 3641 CDR-L1

Arg Ala Ser Gln Asn Ile Asn Asn Phe Leu Asn
                                    10
<400> 16

<210> Sequence Identifier No.17
<211> 11
<212> PRT
<213> Artificial Sequence
<220>
<221> DOMAIN
<222> (1)...(11)
<223> 3641 CDR-L2

Leu Leu Ile Tyr Gly Ser Ser Ile Leu Gln Ser
                                        10
<400> 17


<210> Sequence Identifier No.18
<211> 8
<212> PRT
<213> Artificial Sequence
<220>
<221> DOMAIN
<222> (1)...(8)
<223> 3641 CDR-L3

His Gln Val Ser Asp Thr Ser Asp
                                        10
<400> 18


<210> Sequence Identifier No.19
<211> 126
<212> PRT
<213> Artificial Sequence
<220>
<221> DOMAIN
<222> (1)...(126)
<223> 3640 VH

Gln Val Gln Leu Val Glu Ser Gly Gly Gly Leu Val Gln Pro Gly
                                        10
Gly Ser Leu Arg Leu Ser Cys Ala Ala Ser Gly Phe Thr Phe Asn
                    20                                      030
Asn Ser Trp Met Ser Trp Val Arg Gln Ala Pro Gly Lys Gly Leu
                                       040
Glu Trp Val Ser Asn Ile Thr Ser Ser Ser Ser Met Thr His Tyr
                050                                         060
Ala Asp Ser Val Lys Gly Arg Phe Thr Ile Ser Arg Asp Asn Ser
                                       070
Lys Asn Thr Leu Tyr Leu Gln Met Asn Ser Leu Arg Ala Glu Asp
                080                                         090
Thr Ala Val Tyr Tyr Cys Ala Arg Lys Gly Met Ser Phe Tyr Asn
                                       100
Asn Lys Tyr Ala Lys Ser Met Phe Asp Leu Trp Gly Gln Gly Thr
                110                                         120
Leu Val Thr Val Ser Ser
<400> 19


<210> Sequence Identifier No.20
<211> 110
<212> PRT
<213> Artificial Sequence

<220>
<221> DOMAIN
<222> (1)...(110)
<223> 3640 VL

Asp Ile Glu Leu Thr Gln Pro Pro Ser Val Ser Val Ala Pro Gly
                                        010
Gln Thr Ala Arg Ile Ser Cys Ser Gly Asp Asn Leu Gly Lys Lys
                    020                                     030
Tyr Val His Trp Tyr Gln Gln Lys Pro Gly Gln Ala Pro Val Leu
                                        040
Val Ile Tyr Tyr Asp Ser Lys Arg Pro Ser Gly Ile Pro Glu Arg
                    050                                     060
Phe Ser Gly Ser Asn Ser Gly Asn Thr Ala Thr Leu Thr Ile Ser
                                        070
Gly Thr Gln Ala Glu Asp Glu Ala Asp Tyr Tyr Cys Ser Ala Trp
                    080                                     090
Thr Thr Thr Met Met His Ile Val Phe Gly Gly Gly Thr Lys Leu
                                        100
Thr Val Leu Gly Gln
                    110
<400> 20


<210> Sequence Identifier No.21
<211> 122
<212> PRT
<213> Artificial Sequence
<220>
<221> DOMAIN
<222> (1)...(122)
<223> 3639 VH

Gln Val Gln Leu Val Glu Ser Gly Gly Gly Leu Val Gln Pro Gly
                                        10
Gly Ser Leu Arg Leu Ser Cys Ala Ala Ser Gly Phe Thr Phe Ser
                    20                                      30
Asn Tyr Tyr Leu His Trp Val Arg Gln Ala Pro Gly Lys Gly Leu
                                        40
Glu Trp Val Ser Gly Ile Ser Tyr Ser Gly Ser Ser Thr Tyr Tyr
                    50                                      60
Ala Asp Ser Val Lys Gly Arg Phe Thr Ile Ser Arg Asp Asn Ser
                                        70
Lys Asn Thr Leu Tyr Leu Gln Met Asn Ser Leu Arg Ala Glu Asp
                    80                                      90
Thr Ala Val Tyr Tyr Cys Ala Arg Phe Tyr Gln Arg Arg Gln Ile
                                        100
Pro His Gly Tyr Asp Val Trp Gly Gln Gly Thr Leu Val Thr Val
                    110                                     120
Ser Ser
<400> 21


<210> Sequence Identifier No.22
<211> 110
<212> PRT
<213> Artificial Sequence
<220>
<221> DOMAIN
<222> (1)...(110)
<223> 3639 VL

Asp Ile Glu Leu Thr Gln Pro Pro Ser Val Ser Val Ala Pro Gly
10
Gln Thr Ala Arg Ile Ser Cys Ser Gly Asp Asn Ile Gly Asp Tyr
20 30
Tyr Val His Trp Tyr Gln Gln Lys Pro Gly Gln Ala Pro Val Leu
40
Val Ile Tyr Arg Asp Ser Asn Arg Pro Ser Gly Ile Pro Glu Arg
50 60
Phe Ser Gly Ser Asn Ser Gly Asn Thr Ala Thr Leu Thr Ile Ser
70
Gly Thr Gln Ala Glu Asp Glu Ala Asp Tyr Tyr Cys Gly Ser Tyr
80 90
Asp Met Pro Phe Gly Gly Thr Val Phe Gly Gly Gly Thr Lys Leu
100
Thr Val Leu Gly Gln
                110
<400> 22

<210> Sequence Identifier No.23
<211> 126
<212> PRT
<213> Artificial Sequence
<220>
<221> DOMAIN
<222> (1)...(126)
<223> 3641 VH

Gln Val Gln Leu Val Gln Ser Gly Ala Glu Val Lys Lys Pro Gly
010
Ser Ser Val Lys Val Ser Cys Lys Ala Ser Gly Gly Thr Phe Ser
020 030
Thr Tyr Ala Ile Ser Trp Val Arg Gln Ala Pro Gly Gln Gly Leu
040
Glu Trp Met Gly Arg Ile Ile Pro Ile Phe Gly Thr Ala Asn Tyr
050 060
Ala Gln Lys Phe Gln Gly Arg Val Thr Ile Thr Ala Asp Glu Ser
070
Thr Ser Thr Ala Tyr Met Glu Leu Ser Ser Leu Arg Ser Glu Asp
080 090
Thr Ala Val Tyr Tyr Cys Ala Arg Ser Ser Tyr Thr Ile Leu Thr
100
Thr Tyr Tyr Asn Tyr Asn Ala Met Asp Asn Trp Gly Gln Gly Thr
110 120
Leu Val Thr Val Ser Ser
<400> 23

<210> Sequence Identifier No.24
<211> 109
<212> PRT
<213> Artificial Sequence
<220>
<221> DOMAIN
<222> (1)...(109)
<223> 3641 VL

Asp Ile Gln Met Thr Gln Ser Pro Ser Ser Leu Ser Ala Ser Val
010
Gly Asp Arg Val Thr Ile Thr Cys Arg Ala Ser Gln Asn Ile Asn
020 030
Asn Phe Leu Asn Trp Tyr Gln Gln Lys Pro Gly Lys Ala Pro Lys
040
Leu Leu Ile Tyr Gly Ser Ser Ile Leu Gln Ser Gly Val Pro Ser
050 060
Arg Phe Ser Gly Ser Gly Ser Gly Thr Asp Phe Thr Leu Thr Ile
070
Ser Ser Leu Gln Pro Glu Asp Phe Ala Val Tyr Tyr Cys His Gln
080 090
Val Ser Asp Thr Ser Asp Thr Phe Gly Gln Gly Thr Lys Val Glu
100
Ile Lys Arg Thr
<400> 24

<210> Sequence Identifier No.25
<211> 228
<212> PRT
<213> Artificial Sequence
<220>
<221> DOMAIN
<222> (1)...(228)
<223> 3640 VH CH1
Gln Val Gln Leu Val Glu Ser Gly Gly Gly Leu Val Gln Pro Gly
10
Gly Ser Leu Arg Leu Ser Cys Ala Ala Ser Gly Phe Thr Phe Asn
20 030
Asn Ser Trp Met Ser Trp Val Arg Gln Ala Pro Gly Lys Gly Leu
040
Glu Trp Val Ser Asn Ile Thr Ser Ser Ser Ser Met Thr His Tyr
050 060
Ala Asp Ser Val Lys Gly Arg Phe Thr Ile Ser Arg Asp Asn Ser
070
Lys Asn Thr Leu Tyr Leu Gln Met Asn Ser Leu Arg Ala Glu Asp
080 090
Thr Ala Val Tyr Tyr Cys Ala Arg Lys Gly Met Ser Phe Tyr Asn
100
Asn Lys Tyr Ala Lys Ser Met Phe Asp Leu Trp Gly Gln Gly Thr
110 120
Leu Val Thr Val Ser Ser Ala Ser Thr Lys Gly Pro Ser Val Phe
130
Pro Leu Ala Pro Ser Ser Lys Ser Thr Ser Gly Gly Thr Ala Ala
140 150
Leu Gly Cys Leu Val Lys Asp Tyr Phe Pro Glu Pro Val Thr Val
160
Ser Trp Asn Ser Gly Ala Leu Thr Ser Gly Val His Thr Phe Pro
170 180
Ala Val Leu Gln Ser Ser Gly Leu Tyr Ser Leu Ser Ser Val Val
190
Thr Val Pro Ser Ser Ser Leu Gly Thr Gln Thr Tyr Ile Cys Asn
200 210
Val Asn His Lys Pro Ser Asn Thr Lys Val Asp Lys Lys Val Glu
220
Pro Lys Ser

<400> 25

<210> Sequence Identifier No.26
<211> 213
<212> PRT
<213> Artificial Sequence
<220>
<221> DOMAIN
<222> (1)...(213)
<223> 3640 VL CL

Asp Ile Glu Leu Thr Gln Pro Pro Ser Val Ser Val Ala Pro Gly
                                        010
Gln Thr Ala Arg Ile Ser Cys Ser Gly Asp Asn Leu Gly Lys Lys
                    020                                     030
Tyr Val His Trp Tyr Gln Gln Lys Pro Gly Gln Ala Pro Val Leu
                                        040
Val Ile Tyr Tyr Asp Ser Lys Arg Pro Ser Gly Ile Pro Glu Arg
                    050                                     060
Phe Ser Gly Ser Asn Ser Gly Asn Thr Ala Thr Leu Thr Ile Ser
                                        070
Gly Thr Gln Ala Glu Asp Glu Ala Asp Tyr Tyr Cys Ser Ala Trp
                    080                                     090
Thr Thr Thr Met Met His Ile Val Phe Gly Gly Gly Thr Lys Leu
                                        100
Thr Val Leu Gly Gln Pro Lys Ala Ala Pro Ser Val Thr Leu Phe
                    110                                     120
Pro Pro Ser Ser Glu Glu Leu Gln Ala Asn Lys Ala Thr Leu Val
                                        130
Cys Leu Ile Ser Asp Phe Tyr Pro Gly Ala Val Thr Val Ala Trp
                    140                                     150
Lys Ala Asp Ser Ser Pro Val Lys Ala Gly Val Glu Thr Thr Thr
                                        160
Pro Ser Lys Gln Ser Asn Asn Lys Tyr Ala Ala Ser Ser Tyr Leu
                    170                                     180
Ser Leu Thr Pro Glu Gln Trp Lys Ser His Arg Ser Tyr Ser Cys
                                        190
Gln Val Thr His Glu Gly Ser Thr Val Glu Lys Thr Val Ala Pro
                    200                                     210
Thr Glu Ala
<400> 26


<210> Sequence Identifier No.27
<211> 224
<212> PRT
<213> Artificial Sequence
<220>
<221> DOMAIN
<222> (1)...(224)
<223> 3639 VH CH1

Gln Val Gln Leu Val Glu Ser Gly Gly Gly Leu Val Gln Pro Gly
                                        10
Gly Ser Leu Arg Leu Ser Cys Ala Ala Ser Gly Phe Thr Phe Ser
                    20                                      30
Asn Tyr Tyr Leu His Trp Val Arg Gln Ala Pro Gly Lys Gly Leu
                                        40
Glu Trp Val Ser Gly Ile Ser Tyr Ser Gly Ser Ser Thr Tyr Tyr
                    50                                      60
Ala Asp Ser Val Lys Gly Arg Phe Thr Ile Ser Arg Asp Asn Ser
                                        70
Lys Asn Thr Leu Tyr Leu Gln Met Asn Ser Leu Arg Ala Glu Asp

80                                                    90
Thr Ala Val Tyr Tyr Cys Ala Arg Phe Tyr Gln Arg Arg Gln Ile
                                        100
Pro His Gly Tyr Asp Val Trp Gly Gln Gly Thr Leu Val Thr Val
                110                                     120
Ser Ser Ala Ser Thr Lys Gly Pro Ser Val Phe Pro Leu Ala Pro
                                        130
Ser Ser Lys Ser Thr Ser Gly Gly Thr Ala Ala Leu Gly Cys Leu
                140                                     150
Val Lys Asp Tyr Phe Pro Glu Pro Val Thr Val Ser Trp Asn Ser
                                        160
Gly Ala Leu Thr Ser Gly Val His Thr Phe Pro Ala Val Leu Gln
                170                                     180
Ser Ser Gly Leu Tyr Ser Leu Ser Ser Val Val Thr Val Pro Ser
                                        190
Ser Ser Leu Gly Thr Gln Thr Tyr Ile Cys Asn Val Asn His Lys
                200                                     210
Pro Ser Asn Thr Lys Val Asp Lys Lys Val Glu Pro Lys Ser
                                        220
<400> 27


<210> Sequence Identifier No.28
<211> 213
<212> PRT
<213> Artificial Sequence
<220>
<221> DOMAIN
<222> (1)...(213)
<223> 3639 VL CL

Asp Ile Glu Leu Thr Gln Pro Pro Ser Val Ser Val Ala Pro Gly
                                        10
Gln Thr Ala Arg Ile Ser Cys Ser Gly Asp Asn Ile Gly Asp Tyr
                20                                      30
Tyr Val His Trp Tyr Gln Gln Lys Pro Gly Gln Ala Pro Val Leu
                                        40
Val Ile Tyr Arg Asp Ser Asn Arg Pro Ser Gly Ile Pro Glu Arg
                50                                      60
Phe Ser Gly Ser Asn Ser Gly Asn Thr Ala Thr Leu Thr Ile Ser
                                        70
Gly Thr Gln Ala Glu Asp Glu Ala Asp Tyr Tyr Cys Gly Ser Tyr
                80                                      90
Asp Met Pro Phe Gly Gly Thr Val Phe Gly Gly Gly Thr Lys Leu
                                        100
Thr Val Leu Gly Gln Pro Lys Ala Ala Pro Ser Val Thr Leu Phe
                110                                     120
Pro Pro Ser Ser Glu Glu Leu Gln Ala Asn Lys Ala Thr Leu Val
                                        130
Cys Leu Ile Ser Asp Phe Tyr Pro Gly Ala Val Thr Val Ala Trp
                140                                     150
Lys Ala Asp Ser Ser Pro Val Lys Ala Gly Val Glu Thr Thr Thr
                                        160
Pro Ser Lys Gln Ser Asn Asn Lys Tyr Ala Ala Ser Ser Tyr Leu
                170                                     180
Ser Leu Thr Pro Glu Gln Trp Lys Ser His Arg Ser Tyr Ser Cys
                                        190
Gln Val Thr His Glu Gly Ser Thr Val Glu Lys Thr Val Ala Pro
                200                                     210
Thr Glu Ala
<400> 28

<210> Sequence Identifier No.29
<211> 228
<212> PRT
<213> Artificial Sequence
<220>
<221> DOMAIN
<222> (1)...(228)
<223> 3641 VH CH

Gln Val Gln Leu Val Gln Ser Gly Ala Glu Val Lys Lys Pro Gly
                                        010
Ser Ser Val Lys Val Ser Cys Lys Ala Ser Gly Gly Thr Phe Ser
                020                                     030
Thr Tyr Ala Ile Ser Trp Val Arg Gln Ala Pro Gly Gln Gly Leu
                                        040
Glu Trp Met Gly Arg Ile Ile Pro Ile Phe Gly Thr Ala Asn Tyr
                050                                     060
Ala Gln Lys Phe Gln Gly Arg Val Thr Ile Thr Ala Asp Glu Ser
                                        070
Thr Ser Thr Ala Tyr Met Glu Leu Ser Ser Leu Arg Ser Glu Asp
                080                                     090
Thr Ala Val Tyr Tyr Cys Ala Arg Ser Ser Tyr Thr Ile Leu Thr
                                        100
Thr Tyr Tyr Asn Tyr Asn Ala Met Asp Asn Trp Gly Gln Gly Thr
                110                                     120
Leu Val Thr Val Ser Ser Ala Ser Thr Lys Gly Pro Ser Val Phe
                                        130
Pro Leu Ala Pro Ser Ser Lys Ser Thr Ser Gly Gly Thr Ala Ala
                140                                     150
Leu Gly Cys Leu Val Lys Asp Tyr Phe Pro Glu Pro Val Thr Val
                                        160
Ser Trp Asn Ser Gly Ala Leu Thr Ser Gly Val His Thr Phe Pro
                170                                     180
Ala Val Leu Gln Ser Ser Gly Leu Tyr Ser Leu Ser Ser Val Val
                                        190
Thr Val Pro Ser Ser Ser Leu Gly Thr Gln Thr Tyr Ile Cys Asn
                200                                     210
Val Asn His Lys Pro Ser Asn Thr Lys Val Asp Lys Lys Val Glu
                                        220
Pro Lys Ser

<400> 29


<210> Sequence Identifier No.30
<211> 214
<212> PRT
<213> Artificial Sequence
<220>
<221> DOMAIN
<222> (1)...(214)
<223> 3641 VL CL

Asp Ile Gln Met Thr Gln Ser Pro Ser Ser Leu Ser Ala Ser Val
                                        010
Gly Asp Arg Val Thr Ile Thr Cys Arg Ala Ser Gln Asn Ile Asn
                020                                     030
Asn Phe Leu Asn Trp Tyr Gln Gln Lys Pro Gly Lys Ala Pro Lys

                                        040
Leu Leu Ile Tyr Gly Ser Ser Ile Leu Gln Ser Gly Val Pro Ser
                050                                     060
Arg Phe Ser Gly Ser Gly Ser Gly Thr Asp Phe Thr Leu Thr Ile
                                        070
Ser Ser Leu Gln Pro Glu Asp Phe Ala Val Tyr Tyr Cys His Gln
                080                                     090
Val Ser Asp Thr Ser Asp Thr Phe Gly Gln Gly Thr Lys Val Glu
                                        100
Ile Lys Arg Thr Val Ala Ala Pro Ser Val Phe Ile Phe Pro Pro
                110                                     120
Ser Asp Glu Gln Leu Lys Ser Gly Thr Ala Ser Val Val Cys Leu
                                        130
Leu Asn Asn Phe Tyr Pro Arg Glu Ala Lys Val Gln Trp Lys Val
                140                                     150
Asp Asn Ala Leu Gln Ser Gly Asn Ser Gln Glu Ser Val Thr Glu
                                        160
Gln Asp Ser Lys Asp Ser Thr Tyr Ser Leu Ser Ser Thr Leu Thr
                170                                     180
Leu Ser Lys Ala Asp Tyr Glu Lys His Lys Val Tyr Ala Cys Glu
                                        190
Val Thr His Gln Gly Leu Ser Ser Pro Val Thr Lys Ser Phe Asn
                200                                     210
Arg Gly Glu Ala

<400> 30

SEQUENCE IDENTIFIER

<110> Bayer Schering Pharma Aktiengesellschaft, et al

<120> Anti ephB4 antibody fragments
<130> 53475AEP

<160> 30
<170> PatentIn Ver. 2.1

<210> 1
<211> 10
<212> PRT
<213> Artificial Sequence

<220>
<223> Artificial Sequence

<220>
<221> DOMAIN
<222> (1)...(10)
<223> 3640 CDR-H1
<400> 1
Gly Phe Thr Phe Asn Asn Ser Trp Met Ser
                                    10

<210> 2
<211> 20
<212> PRT
<213> Artificial Sequence

<220>
<223> Artificial Sequence

<220>
<221> DOMAIN
<222> (1)...(20)
<223> 3640 CDR-H2
<400> 2
Trp Val Ser Asn Ile Thr Ser Ser Ser Ser Met Thr His Tyr Ala Asp
                                                10
Ser Val Lys Gly
                20

<210> 3
<211> 17
<212> PRT
<213> Artificial Sequence

<220>

<223> Artificial Sequence

<220>
<221> DOMAIN
<222> (1)...(17)
<223> 3640 CDR-H3
<400> 3
Lys Gly Met Ser Phe Tyr Asn Asn Lys Tyr Ala Lys Ser Met Phe Asp
                                                10
Leu

<210> 4
<211> 11
<212> PRT
<213> Artificial Sequence

<220>
<223> Artificial Sequence

<220>
<221> DOMAIN
<222> (1)...(11)
<223> 3640 CDR-L1
<400> 4
Ser Gly Asp Asn Leu Gly Lys Lys Tyr Val His
                                                10

<210> 5
<211> 11
<212> PRT
<213> Artificial Sequence

<220>
<223> Artificial Sequence

<220>
<221> DOMAIN
<222> (1)...(11)
<223> 3640 CDR-L2
<400> 5
Leu Val Ile Tyr Tyr Asp Ser Lys Arg Pro Ser
                                                10

<210> 6
<211> 10
<212> PRT
<213> Artificial Sequence

<220>
<223> Artificial Sequence

<220>
<221> DOMAIN
<222> (1)...(10)
<223> 3640 CDR-L3
<400> 6
Ser Ala Trp Thr Thr Thr Met Met His Ile
                                    10

<210> 7
<211> 10
<212> PRT
<213> Artificial Sequence

<220>
<223> Artificial Sequence

<220>
<221> DOMAIN
<222> (1)...(10)
<223> 3639 CDR-H1
<400> 7
Gly Phe Thr Phe Ser Asn Tyr Tyr Leu His
                                    10

<210> 8
<211> 17
<212> PRT
<213> Artificial Sequence

<220>
<223> Artificial Sequence

<220>
<221> DOMAIN
<222> (1)...(17)
<223> 3639 CDR-H2
<400> 8
Gly Ile Ser Tyr Ser Gly Ser Ser Thr Tyr Tyr Ala Asp Ser Val Lys
                                    10
Gly

<210> 9
<211> 13
<212> PRT
<213> Artificial Sequence

<220>
<223> Artificial Sequence

<220>
<221> DOMAIN
<222> (1)...(13)
<223> 3639 CDR-H3
<400> 9
Phe Tyr Gln Arg Arg Gln Ile Pro His Gly Tyr Asp Val
                                                10

<210> 10
<211> 11
<212> PRT
<213> Artificial Sequence

<220>
<223> Artificial Sequence

<220>
<221> DOMAIN
<222> (1)...(11)
<223> 3639 CDR-L1
<400> 10
Ser Gly Asp Asn Ile Gly Asp Tyr Tyr Val His
                                                10

<210> 11
<211> 11
<212> PRT
<213> Artificial Sequence

<220>
<223> Artificial Sequence

<220>
<221> DOMAIN
<222> (1)...(11)
<223> 3639 CDR-L2
<400> 11
Leu Val Ile Tyr Arg Asp Ser Asn Arg Pro Ser
                                                10

<210> 12
<211> 10
<212> PRT
<213> Artificial Sequence

<220>
<223> Artificial Sequence

<220>
<221> DOMAIN
<222> (1)...(10)
<223> 3639 CDR-L3
<400> 12
Gly Ser Tyr Asp Met Pro Phe Gly Gly Thr
                                    10

<210> 13
<211> 10
<212> PRT
<213> Artificial Sequence

<220>
<223> Artificial Sequence

<220>
<221> DOMAIN
<222> (1)...(10)
<223> 3641 CDR-H1
<400> 13
Gly Gly Thr Phe Ser Thr Tyr Ala Ile Ser
                                    10

<210> 14
<211> 20
<212> PRT
<213> Artificial Sequence

<220>
<223> Artificial Sequence

<220>
<221> DOMAIN
<222> (1)...(20)
<223> 3641 CDR-H2
<400> 14
Trp Met Gly Arg Ile Ile Pro Ile Phe Gly Thr Ala Asn Tyr Ala Gln
                                    10
Lys Phe Gln Gly
            20

<210> 15
<211> 17

<212> PRT
<213> Artificial Sequence

<220>
<223> Artificial Sequence

<220>
<221> DOMAIN
<222> (1)...(17)
<223> 3641 CDR-H3
<400> 15
Ser Ser Tyr Thr Ile Leu Thr Thr Tyr Tyr Asn Tyr Asn Ala Met Asp
                                        10
Asn

<210> 16
<211> 11
<212> PRT
<213> Artificial Sequence

<220>
<223> Artificial Sequence

<220>
<221> DOMAIN
<222> (1)...(11)
<223> 3641 CDR-L1
<400> 16
Arg Ala Ser Gln Asn Ile Asn Asn Phe Leu Asn
                                                10

<210> 17
<211> 11
<212> PRT
<213> Artificial Sequence

<220>
<223> Artificial Sequence

<220>
<221> DOMAIN
<222> (1)...(11)
<223> 3641 CDR-L2
<400> 17
Leu Leu Ile Tyr Gly Ser Ser Ile Leu Gln Ser
                                                10

<210> 18
<211> 8
<212> PRT
<213> Artificial Sequence

<220>
<223> Artificial Sequence

<220>
<221> DOMAIN
<222> (1)...(8)
<223> 3641 CDR-L3
<400> 18
His Gln Val Ser Asp Thr Ser Asp
                                                10

<210> 19
<211> 126
<212> PRT
<213> Artificial Sequence

<220>
<223> Artificial Sequence

<220>
<221> DOMAIN
<222> (1)...(126)
<223> 3640 VH
<400> 19
Gln Val Gln Leu Val Glu Ser Gly Gly Gly Leu Val Gln Pro Gly
                                                10
Gly Ser Leu Arg Leu Ser Cys Ala Ala Ser Gly Phe Thr Phe Asn
            20                                                  030
Asn Ser Trp Met Ser Trp Val Arg Gln Ala Pro Gly Lys Gly Leu
                                                040
Glu Trp Val Ser Asn Ile Thr Ser Ser Ser Ser Met Thr His Tyr
            050                                                 060
Ala Asp Ser Val Lys Gly Arg Phe Thr Ile Ser Arg Asp Asn Ser
                                                070
Lys Asn Thr Leu Tyr Leu Gln Met Asn Ser Leu Arg Ala Glu Asp
            080                                                 090
Thr Ala Val Tyr Tyr Cys Ala Arg Lys Gly Met Ser Phe Tyr Asn
                                                100
Asn Lys Tyr Ala Lys Ser Met Phe Asp Leu Trp Gly Gln Gly Thr
            110                                                 120
Leu Val Thr Val Ser Ser

<210> 20
<211> 110
<212> PRT

<213> Artificial Sequence

<220>
<223> Artificial Sequence

<220>
<221> DOMAIN
<222> (1)...(110)
<223> 3640 VL
<400> 20
Asp Ile Glu Leu Thr Gln Pro Pro Ser Val Ser Val Ala Pro Gly
                                                    010
Gln Thr Ala Arg Ile Ser Cys Ser Gly Asp Asn Leu Gly Lys Lys
    020                                                 030
Tyr Val His Trp Tyr Gln Gln Lys Pro Gly Gln Ala Pro Val Leu
                                                    040
Val Ile Tyr Tyr Asp Ser Lys Arg Pro Ser Gly Ile Pro Glu Arg
    050                                                 060
Phe Ser Gly Ser Asn Ser Gly Asn Thr Ala Thr Leu Thr Ile Ser
                                                    070
Gly Thr Gln Ala Glu Asp Glu Ala Asp Tyr Tyr Cys Ser Ala Trp
            080                                             090
Thr Thr Thr Met Met His Ile Val Phe Gly Gly Gly Thr Lys Leu
                                                    100
Thr Val Leu Gly Gln
            110

<210> 21
<211> 122
<212> PRT
<213> Artificial Sequence

<220>
<223> Artificial Sequence

<220>
<221> DOMAIN
<222> (1)...(122)
<223> 3639 VH
<400> 21
Gln Val Gln Leu Val Glu Ser Gly Gly Gly Leu Val Gln Pro Gly
                                                     10
Gly Ser Leu Arg Leu Ser Cys Ala Ala Ser Gly Phe Thr Phe Ser
                         20
   30
Asn Tyr Tyr Leu His Trp Val Arg Gln Ala Pro Gly Lys Gly Leu
                                                     40
Glu Trp Val Ser Gly Ile Ser Tyr Ser Gly Ser Ser Thr Tyr Tyr
                         50
   60
Ala Asp Ser Val Lys Gly Arg Phe Thr Ile Ser Arg Asp Asn Ser
                                                     70

Lys Asn Thr Leu Tyr Leu Gln Met Asn Ser Leu Arg Ala Glu Asp
                        80
   90
Thr Ala Val Tyr Tyr Cys Ala Arg Phe Tyr Gln Arg Arg Gln Ile
                                                100
Pro His Gly Tyr Asp Val Trp Gly Gln Gly Thr Leu Val Thr Val
                        110
  120
Ser Ser

<210> 22
<211> 110
<212> PRT
<213> Artificial Sequence

<220>
<223> Artificial Sequence

<220>
<221> DOMAIN
<222> (1)...(110)
<223> 3639 VL
<400> 22
Asp Ile Glu Leu Thr Gln Pro Pro Ser Val Ser Val Ala Pro Gly
                                                10
Gln Thr Ala Arg Ile Ser Cys Ser Gly Asp Asn Ile Gly Asp Tyr
            20                                              30
Tyr Val His Trp Tyr Gln Gln Lys Pro Gly Gln Ala Pro Val Leu
                                                 40
Val Ile Tyr Arg Asp Ser Asn Arg Pro Ser Gly Ile Pro Glu Arg
            50                                              60
Phe Ser Gly Ser Asn Ser Gly Asn Thr Ala Thr Leu Thr Ile Ser
                                                 70
Gly Thr Gln Ala Glu Asp Glu Ala Asp Tyr Tyr Cys Gly Ser Tyr
            80                                              90
Asp Met Pro Phe Gly Gly Thr Val Phe Gly Gly Gly Thr Lys Leu
                                                100
Thr Val Leu Gly Gln
            110

<210> 23
<211> 126
<212> PRT
<213> Artificial Sequence

<220>
<223> Artificial Sequence

<220>

<221> DOMAIN
<222> (1)...(126)
<223> 3641 VH
<400> 23
Gln Val Gln Leu Val Gln Ser Gly Ala Glu Val Lys Lys Pro Gly
                                                        010
Ser Ser Val Lys Val Ser Cys Lys Ala Ser Gly Gly Thr Phe Ser
            020                                             030
Thr Tyr Ala Ile Ser Trp Val Arg Gln Ala Pro Gly Gln Gly Leu
                                                        040
Glu Trp Met Gly Arg Ile Ile Pro Ile Phe Gly Thr Ala Asn Tyr
            050                                             060
Ala Gln Lys Phe Gln Gly Arg Val Thr Ile Thr Ala Asp Glu Ser
                                                        070
Thr Ser Thr Ala Tyr Met Glu Leu Ser Ser Leu Arg Ser Glu Asp
            080                                             090
Thr Ala Val Tyr Tyr Cys Ala Arg Ser Ser Tyr Thr Ile Leu Thr
                                                        100
Thr Tyr Tyr Asn Tyr Asn Ala Met Asp Asn Trp Gly Gln Gly Thr
            110                                             120
Leu Val Thr Val Ser Ser

<210> 24
<211> 109
<212> PRT
<213> Artificial Sequence

<220>
<223> Artificial Sequence

<220>
<221> DOMAIN
<222> (1)...(109)
<223> 3641 VL
<400> 24
Asp Ile Gln Met Thr Gln Ser Pro Ser Ser Leu Ser Ala Ser Val
                                                        010
Gly Asp Arg Val Thr Ile Thr Cys Arg Ala Ser Gln Asn Ile Asn
            020                                             030
Asn Phe Leu Asn Trp Tyr Gln Gln Lys Pro Gly Lys Ala Pro Lys
                                                        040
Leu Leu Ile Tyr Gly Ser Ser Ile Leu Gln Ser Gly Val Pro Ser
            050                                             060
Arg Phe Ser Gly Ser Gly Ser Gly Thr Asp Phe Thr Leu Thr Ile
                                                        070
Ser Ser Leu Gln Pro Glu Asp Phe Ala Val Tyr Tyr Cys His Gln
            080                                             090
Val Ser Asp Thr Ser Asp Thr Phe Gly Gln Gly Thr Lys Val Glu
                                                        100
Ile Lys Arg Thr

<210> 25
<211> 228
<212> PRT
<213> Artificial Sequence

<220>
<223> Artificial Sequence

<220>
<221> DOMAIN
<222> (1)...(228)
<223> 3640 VH CH1
<400> 25
Gln Val Gln Leu Val Glu Ser Gly Gly Gly Leu Val Gln Pro Gly
                                                    10
Gly Ser Leu Arg Leu Ser Cys Ala Ala Ser Gly Phe Thr Phe Asn
            20                                                  030
Asn Ser Trp Met Ser Trp Val Arg Gln Ala Pro Gly Lys Gly Leu
                                                    040
Glu Trp Val Ser Asn Ile Thr Ser Ser Ser Ser Met Thr His Tyr
            050                                                 060
Ala Asp Ser Val Lys Gly Arg Phe Thr Ile Ser Arg Asp Asn Ser
                                                    070
Lys Asn Thr Leu Tyr Leu Gln Met Asn Ser Leu Arg Ala Glu Asp
            080                                                 090
Thr Ala Val Tyr Tyr Cys Ala Arg Lys Gly Met Ser Phe Tyr Asn
                                                    100
Asn Lys Tyr Ala Lys Ser Met Phe Asp Leu Trp Gly Gln Gly Thr
            110                                                 120
Leu Val Thr Val Ser Ser Ala Ser Thr Lys Gly Pro Ser Val Phe
                                                    130
Pro Leu Ala Pro Ser Ser Lys Ser Thr Ser Gly Gly Thr Ala Ala
            140                                                 150
Leu Gly Cys Leu Val Lys Asp Tyr Phe Pro Glu Pro Val Thr Val
                                                    160
Ser Trp Asn Ser Gly Ala Leu Thr Ser Gly Val His Thr Phe Pro
            170                                                 180
Ala Val Leu Gln Ser Ser Gly Leu Tyr Ser Leu Ser Ser Val Val
                                        190
Thr Val Pro Ser Ser Ser Leu Gly Thr Gln Thr Tyr Ile Cys Asn
            200                                                 210
Val Asn His Lys Pro Ser Asn Thr Lys Val Asp Lys Lys Val Glu
                                                    220
Pro Lys Ser

<210> 26
<211> 213
<212> PRT
<213> Artificial Sequence

<220>
<223> Artificial Sequence

<220>
<221> DOMAIN
<222> (1)...(213)
<223> 3640 VL CL
<400> 26
Asp Ile Glu Leu Thr Gln Pro Pro Ser Val Ser Val Ala Pro Gly
                                                        010
Gln Thr Ala Arg Ile Ser Cys Ser Gly Asp Asn Leu Gly Lys Lys
    020                                                     030
Tyr Val His Trp Tyr Gln Gln Lys Pro Gly Gln Ala Pro Val Leu
                                                        040
Val Ile Tyr Tyr Asp Ser Lys Arg Pro Ser Gly Ile Pro Glu Arg
    050                                                     060
Phe Ser Gly Ser Asn Ser Gly Asn Thr Ala Thr Leu Thr Ile Ser
                                                        070
Gly Thr Gln Ala Glu Asp Glu Ala Asp Tyr Tyr Cys Ser Ala Trp
            080                                                 090
Thr Thr Thr Met Met His Ile Val Phe Gly Gly Gly Thr Lys Leu
                                                        100
Thr Val Leu Gly Gln Pro Lys Ala Ala Pro Ser Val Thr Leu Phe
            110                                                 120
Pro Pro Ser Ser Glu Glu Leu Gln Ala Asn Lys Ala Thr Leu Val
                                                        130
Cys Leu Ile Ser Asp Phe Tyr Pro Gly Ala Val Thr Val Ala Trp
            140                                                 150
Lys Ala Asp Ser Ser Pro Val Lys Ala Gly Val Glu Thr Thr Thr
                                                        160
Pro Ser Lys Gln Ser Asn Asn Lys Tyr Ala Ala Ser Ser Tyr Leu
            170                                                 180
Ser Leu Thr Pro Glu Gln Trp Lys Ser His Arg Ser Tyr Ser Cys
                                                        190
Gln Val Thr His Glu Gly Ser Thr Val Glu Lys Thr Val Ala Pro
    200                                                     210
Thr Glu Ala

<210> 27
<211> 224
<212> PRT
<213> Artificial Sequence

<220>
<223> Artificial Sequence

<220>
<221> DOMAIN
<222> (1)...(224)
<223> 3639 VH CH1
<400> 27

Gln Val Gln Leu Val Glu Ser Gly Gly Gly Leu Val Gln Pro Gly
10
Gly Ser Leu Arg Leu Ser Cys Ala Ala Ser Gly Phe Thr Phe Ser
20
30
Asn Tyr Tyr Leu His Trp Val Arg Gln Ala Pro Gly Lys Gly Leu
40
Glu Trp Val Ser Gly Ile Ser Tyr Ser Gly Ser Ser Thr Tyr Tyr
50
60
Ala Asp Ser Val Lys Gly Arg Phe Thr Ile Ser Arg Asp Asn Ser
70
Lys Asn Thr Leu Tyr Leu Gln Met Asn Ser Leu Arg Ala Glu Asp
80
90
Thr Ala Val Tyr Tyr Cys Ala Arg Phe Tyr Gln Arg Arg Gln Ile
100
Pro His Gly Tyr Asp Val Trp Gly Gln Gly Thr Leu Val Thr Val
110
120
Ser Ser Ala Ser Thr Lys Gly Pro Ser Val Phe Pro Leu Ala Pro
130
Ser Ser Lys Ser Thr Ser Gly Gly Thr Ala Ala Leu Gly Cys Leu
140 150
Val Lys Asp Tyr Phe Pro Glu Pro Val Thr Val Ser Trp Asn Ser
160
Gly Ala Leu Thr Ser Gly Val His Thr Phe Pro Ala Val Leu Gln
170 180
Ser Ser Gly Leu Tyr Ser Leu Ser Ser Val Val Thr Val Pro Ser
190
Ser Ser Leu Gly Thr Gln Thr Tyr Ile Cys Asn Val Asn His Lys
200
210
Pro Ser Asn Thr Lys Val Asp Lys Lys Val Glu Pro Lys Ser
220

<210> 28
<211> 213
<212> PRT
<213> Artificial Sequence

<220>
<223> Artificial Sequence

<220>
<221> DOMAIN
<222> (1)...(213)
<223> 3639 VL CL

<400> 28
Asp Ile Glu Leu Thr Gln Pro Pro Ser Val Ser Val Ala Pro Gly
10

Gln Thr Ala Arg Ile Ser Cys Ser Gly Asp Asn Ile Gly Asp Tyr
20 30
Tyr Val His Trp Tyr Gln Gln Lys Pro Gly Gln Ala Pro Val Leu
40
Val Ile Tyr Arg Asp Ser Asn Arg Pro Ser Gly Ile Pro Glu Arg
50 60
Phe Ser Gly Ser Asn Ser Gly Asn Thr Ala Thr Leu Thr Ile Ser
70
Gly Thr Gln Ala Glu Asp Glu Ala Asp Tyr Tyr Cys Gly Ser Tyr
80 90
Asp Met Pro Phe Gly Gly Thr Val Phe Gly Gly Gly Thr Lys Leu
100
Thr Val Leu Gly Gln Pro Lys Ala Ala Pro Ser Val Thr Leu Phe
110 120
Pro Pro Ser Ser Glu Glu Leu Gln Ala Asn Lys Ala Thr Leu Val
130
Cys Leu Ile Ser Asp Phe Tyr Pro Gly Ala Val Thr Val Ala Trp
140 150
Lys Ala Asp Ser Ser Pro Val Lys Ala Gly Val Glu Thr Thr Thr
160
Pro Ser Lys Gln Ser Asn Asn Lys Tyr Ala Ala Ser Ser Tyr Leu
170 180
Ser Leu Thr Pro Glu Gln Trp Lys Ser His Arg Ser Tyr Ser Cys
190
Gln Val Thr His Glu Gly Ser Thr Val Glu Lys Thr Val Ala Pro
200 210
Thr Glu Ala

<210> 29
<211> 228
<212> PRT
<213> Artificial Sequence

<220>
<223> Artificial Sequence

<220>
<221> DOMAIN
<222> (1)...(228)
<223> 3641 VH CH
<400> 29
Gln Val Gln Leu Val Gln Ser Gly Ala Glu Val Lys Lys Pro Gly
010
Ser Ser Val Lys Val Ser Cys Lys Ala Ser Gly Gly Thr Phe Ser
020 030
Thr Tyr Ala Ile Ser Trp Val Arg Gln Ala Pro Gly Gln Gly Leu
040
Glu Trp Met Gly Arg Ile Ile Pro Ile Phe Gly Thr Ala Asn Tyr
050 060
Ala Gln Lys Phe Gln Gly Arg Val Thr Ile Thr Ala Asp Glu Ser
070
Thr Ser Thr Ala Tyr Met Glu Leu Ser Ser Leu Arg Ser Glu Asp

080                                                    090
Thr Ala Val Tyr Tyr Cys Ala Arg Ser Ser Tyr Thr Ile Leu Thr
                                                100
Thr Tyr Tyr Asn Tyr Asn Ala Met Asp Asn Trp Gly Gln Gly Thr
            110                                         120
Leu Val Thr Val Ser Ser Ala Ser Thr Lys Gly Pro Ser Val Phe
                                                130
Pro Leu Ala Pro Ser Ser Lys Ser Thr Ser Gly Gly Thr Ala Ala
            140                                         150
Leu Gly Cys Leu Val Lys Asp Tyr Phe Pro Glu Pro Val Thr Val
                                                160
Ser Trp Asn Ser Gly Ala Leu Thr Ser Gly Val His Thr Phe Pro
            170                                         180
Ala Val Leu Gln Ser Ser Gly Leu Tyr Ser Leu Ser Ser Val Val
                                                190
Thr Val Pro Ser Ser Ser Leu Gly Thr Gln Thr Tyr Ile Cys Asn
            200                                         210
Val Asn His Lys Pro Ser Asn Thr Lys Val Asp Lys Lys Val Glu
                                    220
Pro Lys Ser

<210> 30
<211> 214
<212> PRT
<213> Artificial Sequence

<220>
<223> Artificial Sequence

<220>
<221> DOMAIN
<222> (1)...(214)
<223> 3641 VL CL

<400> 30
Asp Ile Gln Met Thr Gln Ser Pro Ser Ser Leu Ser Ala Ser Val
                                                010
Gly Asp Arg Val Thr Ile Thr Cys Arg Ala Ser Gln Asn Ile Asn
            020                                         030
Asn Phe Leu Asn Trp Tyr Gln Gln Lys Pro Gly Lys Ala Pro Lys
                                                040
Leu Leu Ile Tyr Gly Ser Ser Ile Leu Gln Ser Gly Val Pro Ser
            050                                         060
Arg Phe Ser Gly Ser Gly Ser Gly Thr Asp Phe Thr Leu Thr Ile
                                                070
Ser Ser Leu Gln Pro Glu Asp Phe Ala Val Tyr Tyr Cys His Gln
            080                                         090
Val Ser Asp Thr Ser Asp Thr Phe Gly Gln Gly Thr Lys Val Glu
                                                100
Ile Lys Arg Thr Val Ala Ala Pro Ser Val Phe Ile Phe Pro Pro
            110                                         120

Ser Asp Glu Gln Leu Lys Ser Gly Thr Ala Ser Val Val Cys Leu
                                                130
Leu Asn Asn Phe Tyr Pro Arg Glu Ala Lys Val Gln Trp Lys Val
            140                                             150
Asp Asn Ala Leu Gln Ser Gly Asn Ser Gln Glu Ser Val Thr Glu
                                                160
Gln Asp Ser Lys Asp Ser Thr Tyr Ser Leu Ser Ser Thr Leu Thr
            170                                             180
Leu Ser Lys Ala Asp Tyr Glu Lys His Lys Val Tyr Ala Cys Glu
                                                190
Val Thr His Gln Gly Leu Ser Ser Pro Val Thr Lys Ser Phe Asn
            200                                             210
Arg Gly Glu Ala

## Claims

1. Antibody fragment, specifically recognising the EphB4 receptor comprising a CDR of a heavy chain comprising CDR-H1, CDR-H2 and CDR-H3 and a light chain comprising CDR-L1, CDR-L2 and CDR-L3 wherein one of the heavy chains comprising the CDR-H1 selected from group:
Seq Id No 1,
Seq Id No 7,
Seq Id No 13
or a modification thereof
and
the CDR-H2 selected from the group:
Seq Id No 2,
Seq Id No 8,
Seq Id No 14
and the CDR-H3 selected from the group:
Seq Id No 3,
Seq Id No 9,
Seq Id No 15
or modifications thereof
and / or
wherein one of the light chains comprising
the CDR-L1 selected from the group
Seq Id No 4,
Seq Id No 10,
Seq Id No 16
or modifications thereof
and
the CDR-L2 selected from the group
Seq Id No 5,
Seq Id No 11,
Seq Id No 17
or modifications thereof
and
the CDR-L3 selected from the group
Seq Id No 6,
Seq Id No 12,
Seq Id No 18
and modifications thereof.

2. Antibody fragment according to claim 1, specifically recognising the EphB4 receptor comprising a complementarity determining region (CDR) of
a heavy chain
and
a light chain
wherein the CDR of the heavy chain (CDR-H) comprises the sequence of the aminoacids of the
Seq Id No 1 (3640 CDR-H1) and
Seq Id No 2 (3640 CDR-H2) and
Seq Id No 3 (3640 CDR-H3)
or modifications thereof
or the
Seq Id No 7 (3639 CDR-H1) and
Seq Id No 8 (3639 CDR-H2) and
Seq Id No 9 (3639 CDR-H3)
or modifications thereof
or the
Seq Id No 13 (3641 CDR-H1) and
Seq Id No 14 (3641 CDR-H2) and
Seq Id No 15 (3641 CDR-H3)
or modifications thereof
and / or
wherein the CDR of the light chain (CDR-L) comprises the sequence of the aminoacids of the
Seq Id No 4 (3640 CDR-L1) and
Seq Id No 5 (3640 CDR-L2) and
Seq Id No 6 (3640 CDR-L3)
or modifications thereof
or the
Seq Id No 10 (3639 CDR-L1) and
Seq Id No 11 (3639 CDR-L2) and
Seq Id No 12 (3639 CDR-L3)
or modifications thereof
or the
Seq Id No 16 (3641 CDR-L1) and
Seq Id No 17 (3641 CDR-L2) and
Seq Id No 18 (3641 CDR-L3)
or modifications thereof.

3. Antibody fragment according to claim 1, specifically recognising the EphB4 receptor comprising a heavy chain selected from the group:
Seq Id No 19 (3640 variable domain of CDR-H) and
Seq Id No 21 (3639 variable domain of CDR-H) and
Seq Id No 23 (3641 variable domain of CDR-H)
and modifications thereof,
and a light chain selected from the group:
Seq Id No 20 (3640 variable domain of CDR-H) and
Seq Id No 22 (3639 variable domain of CDR-H) and
Seq Id No 24 (3641 variable domain of CDR-H)
and modifications thereof.

4. An antibody fragment according to claim 3, wherein the antibody fragment specifically recognises the EphB4 receptor comprising
Seq Id No 19 (variable domain 3640 CDR-H) and
Seq Id No 20 (variable domain 3640 CDR-L)
or
Seq Id No 21 (variable domain 3639 CDR-H) and
Seq Id No 22 (variable domain 3639 CDR-L)
or
Seq Id No 23 (variable domain 3641 CDR-H) and
Seq Id No 24 (variable domain 3641 CDR-L)
or modifications thereof.

5. Antibody fragment according to any one of the proceeding claims, wherein the antibody fragment is a monomer or a dimer.

6. Antibody fragment according to claim 1 or 3, specifically recognising the EphB4 receptor comprising heavy chain (CDR-H/C) selected from the group:
Seq Id No 25 (3640)
Seq Id No 27 (3639)
Seq Id No 29 (3641)
and
a light chain (CDR-UC) selected from the group:
Seq Id No 26 (3640)
Seq Id No 28 (3639)
Seq Id No 30 (3641)
and modifications thereof.

7. Antibody fragment according to any one of the claims 1, 3 and 4, specifically recognising the EphB4 receptor comprising
Seq Id No 25 (3640 CDR-H/C)
Seq Id No 26 (3640 CDR-UC)
or
Seq Id No 27 (3639 CDR-H/C)
Seq Id No 28 (3639 CDR-UC)
or
Seq Id No 29 (3641 CDR-H/C)
Seq Id No 30 (3641 CDR-UC)
or modifications thereof.

8. Antibody fragment which exhibits a substantially equivalent antigen binding to the EphB4 receptor comparable to an antibody fragment according to any one of the proceeding claims 1 and 3 to 7 or
an antibody fragment which binds to the same epitope on the EphB4 receptor like an antibody fragment according to any of the proceeding claims.

9. Antibody fragment according to any one of the proceeding claims wherein the heavy chain C-terminal and the light chain is N-terminal or the heavy chain N-terminal and the light chain is C-terminal.

10. Antibody fragment according to any of the proceeding claims as a medicament.

11. Antibody fragment according to claim 10, wherein the antibody fragment is used in pathological angiogenesis in particular cancer therapy.

12. Antibody fragment according to any of any one of the claims 10 to 11 claims wherein the antibody fragment is administered with a further substance in cancer therapy.
